(19) **Europäisches Patentamt / European Patent Office / Office européen des brevets**

(11) **EP 4 292 607 A1**

(12) **EUROPEAN PATENT APPLICATION**
published in accordance with Art. 153(4) EPC

(43) Date of publication:
**20.12.2023 Bulletin 2023/51**

(21) Application number: **22739080.4**

(22) Date of filing: **13.01.2022**

(51) International Patent Classification (IPC):
**A61K 39/12** (2006.01)   **A61K 39/295** (2006.01)
**A61K 39/39** (2006.01)   **A61P 31/20** (2006.01)
**A61P 35/00** (2006.01)   **A61P 15/00** (2006.01)

(52) Cooperative Patent Classification (CPC):
**A61K 39/12; A61K 39/295; A61K 39/39;**
**A61P 15/00; A61P 31/20; A61P 35/00**

(86) International application number:
**PCT/CN2022/071782**

(87) International publication number:
**WO 2022/152204 (21.07.2022 Gazette 2022/29)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **14.01.2021   CN 202110049777**

(71) Applicant: **Sinocelltech Ltd.**
**Beijing 100176 (CN)**

(72) Inventors:
• **LIU, Yan**
**Beijing 100176 (CN)**

• **HU, Ping**
**Beijing 100176 (CN)**
• **CHANG, Xinrong**
**Beijing 100176 (CN)**

(74) Representative: **Barker Brettell LLP**
**100 Hagley Road**
**Edgbaston**
**Birmingham, West Midlands B16 8QQ (GB)**

Remarks:
The complete document including Reference Table(s) and the Sequence Listing(s) can be downloaded from the EPO website

(54) **STABLE PREPARATION OF HUMAN PAPILLOMAVIRUS VIRUS-LIKE PARTICLE VACCINE**

(57)   Provided is a stable formulation of a human papillomavirus virus-like particle vaccine. The stable formulation is composed of a human papillomavirus virus-like particle, a buffer solution, an osmotic pressure regulator, a surfactant and an aluminum adjuvant, wherein the components of the vaccine comprise HPV virus-like particles assembled by L1 proteins of HPV types 6, 11, 16, 18, 31, 33, 45, 52 and 58, and one or more HPV virus-like particles assembled by L1 proteins of other pathogenic HPV types. The formulation can enhance the stability of the vaccine and prolong the validity period of the vaccine in an aqueous formulation.

Figure 8

EP 4 292 607 A1

**Description**

**CROSS-REFERENCE TO RELATED APPLICATIONS**

This application claims the benefit of Chinese patent application 202110049777.7 filed on January 14, 2021, the contents of which are incorporated herein by reference.

**FIELD**

The invention relates to the field of biological pharmaceutical formulations, in particular to a stable human papillomavirus virus-like particle vaccine formulation.

**BACKGROUND**

Cervical cancer is one of the most common female malignant tumors, with about 500,000 new patients worldwide every year, and the incidence rate is the second among female tumors. More than 95% of cervical cancer is associate with Human Papillomavirus, HPV) infection. In addition to the direct cause of cervical cancer, HPV is also strongly associate with bronchogenic cancer, rectal cancer, oral cancer and skin cancer. In addition, HPV is also the main pathogenic factor causing skin and mucosal warts.

At present, more than 100 HPV types have been found, and different HPV types can cause different diseases. According to its close relationship with cervical cancer, HPV can be divided into high-risk type, suspected carcinogenic type and low-risk type. High risk type and suspected carcinogenic type can induce cancer such as cervical cancer; the high-risk types included types16, 18, 31, 33, 35, 39, 45, 51, 52, 56, 58 and 59. Suspected carcinogenic types include types 26, 53, 66, 68, 73 and 82. The low-risk types are mostly related to genital warts, condyloma acuminatum and other diseases, including types 6, 11, 40, 42, 43, 44, 54, 61, 70, 72, 81 and 89. HPV types 6, 11 and 16 were the subtypes with the highest detection rate in patients with genital lesions.

HPV vaccine is an effective way to block the infection of papillomavirus. Virus-like particle (VLP) vaccine is the most advantageous vaccine form among many vaccine forms. However, the VLP-based HPV vaccine is type specific, i.e., it only shows strong protection against HPV types in the VLP vaccine. To provide broad protection, the development of multivalent HPV vaccines is necessary.

However, prior to administration, the human papillomavirus vaccine formulation undergoes a storage and transportation process during which the antigen undergoes physical and chemical degradation, and these instabilities may reduce the immunogenicity and/or safety of the antigen, and thus a stable formulation is needed to ensure that the antigen still maintains the immunogenicity and safety satisfying prevention purpose just prior to administration.

**SUMMARY**

In one aspect, the present invention provides a stable formulation of multivalent human papillomavirus virus-like particle vaccine for the prevention of HPV-related diseases or infections comprising a plurality of papillomavirus virus-like particles adsorbed on an adjuvant; a physiologically acceptable concentration of a buffer, an osmotic pressure regulator, and optionally a surfactant.

Wherein the human papillomavirus virus-like particles are selected from HPV virus-like particles assembled by L1 proteins of HPV types 6, 11, 16, 18, 31, 33, 45, 52 and 58 respectively; and one or more HPV virus-like particles assembled from L1 proteins of other pathogenic HPV types.

In one embodiment,

the buffer is selected from one or more of citric acid buffer, acetic acid buffer or histidine buffer;
the osmotic pressure regulator is selected from one or more of sodium chloride, sodium phosphate or sodium sulfate;
the surfactant is a polyethoxy ether, preferably polysorbate 80;
the adjuvant is selected from one or more of aluminum hydroxyphosphate ($AlPO_4$), amorphous aluminum hydroxyphosphate sulfate (AAHS), or aluminum hydroxide ($Al(OH)_3$), preferably aluminum hydroxyphosphate ($AlPO_4$).

In one embodiment,

(a) the total concentration of papillomavirus virus-like particles of all types is 40 $\mu$g/mL to 740 $\mu$g/mL;
(b) the concentration of the buffer is 10mM to 26 mM, preferably 10 mM, 18mM or 26 mM;
(c) the concentration of the osmotic pressure regulator is 150mM to 320 mM, preferably 150 mM or 320 mM;
(d) the concentration of the surfactant is 0 to 0.02% by weight;

(e) the concentration of the adjuvant is about 1.0 mg/mL;
(f) the pH of the formulation is 5.9-6.5, preferably 5.9, 6.2 or 6.5.

In one embodiment, the concentration of any single type of papillomavirus virus-like particles included in the multivalent papillomavirus virus-like particles is 40 μg/mL to 120 μg/mL.

In one embodiment, that formulation comprises a total of 0.74mg/mL of papillomavirus virus-like particles of all types, 1.0mg/mL of aluminum phosphate adjuvant, 18mM histidine buff, 320mM sodium chloride, a pH of the formulation solution of 6.2; optionally, polysorbate 80 at a concentration of not more than 0.3mg/mL.

In one embodiment, the one or more other pathogenic HPV types are selected from HPV types 35, 39, 51, 56 and 59.

In one embodiment, wherein at least one of the HPV virus-like particles is a chimeric HPV virus-like particle comprising a chimeric HPV L1 protein; the chimeric HPV L1 protein comprise from that N-terminal to the C-terminal thereof:

a. an N-terminal fragment derived from a papillomavirus L1 protein of a first type, that N-terminal fragment maintaining the immunogenicity of the L1 protein of that type, wherein the papillomavirus of the first type is selected from HPV type 6, 11, 16, 18, 31, 33, 45, 52 and 58 and one or more other pathogenic HPV types; and
b. a C-terminal fragment derived from a second type of papillomavirus L1 protein having better characteristics of expression and solubility compared to L1 proteins of other types;

wherein the chimeric HPV L1 protein has immunogenicity of the first type of papillomavirus L1 protein.

In one embodiment, Said N-terminal fragment is a fragment obtained by truncating the C-terminus of the natural sequence of said L1 protein of the first papilloma virus type at any amino acid position within its α5 region, and a fragment having at least 98% identity therewith; and Said C-terminal fragment is a fragment obtained by truncating the N-terminus of the natural sequence of said L1 protein of the second papilloma virus type at any amino acid position within its α5 region and functional variants resulting from further mutations, deletions and/or additions to the fragment.

In one embodiment, the C-terminal fragment comprises one or more nuclear localization sequences.

In one embodiment, wherein the papilloma L1 protein of the first type is selected from HPV type 6, 11, 16, 18, 31, 35, 39, 45, 51, 52, 56 or 58; preferably, the natural sequence thereof is an amino acid sequence encoded by a coding gene as shown in SEQ ID No: 30, SEQ ID No: 31, SEQ ID No: 32, SEQ ID No: 33, SEQ ID No: 34, SEQ ID No: 35, SEQ ID No: 36, SEQ ID No: 37, SEQ ID No: 38, SEQ ID No: 39, SEQ ID No: 40, or SEQ ID No: 41, respectively;

the papilloma L1 protein of the second type is selected from HPV type 16, 28, 33, 59, or 68 L1 protein;
more preferably, the papilloma L1 protein of the second type is selected from an HPV type 33 or HPV type 59 L1 protein.

In one embodiment, that C-terminal fragment is SEQ ID No: 1; or a fragment thereof having a length of m1 amino acids, preferably a fragment covering amino acids 1-m1 of SEQ ID No:1; wherein m1 is an integer from 8 to 26; or that C-terminal fragment is SEQ ID No: 2; or a fragment thereof having a length of m2 amino acids, preferably a fragment covering amino acids 1-m2 of SEQ ID No:2; wherein m2 is an integer from 13 to 31.

In one embodiment, that C-terminal fragment is SEQ ID No: 3; or a fragment thereof having a length of n amino acids, preferably a fragment covering amino acids 1-n of SEQ ID No:3; wherein n is an integer from 16 to 38.

In one embodiment, the N-terminal fragment of the HPV type 6 L1 protein has 98%, 98.5%, 99%, 99.5%, 99% or 100% identity with a fragment obtained by truncating the C-terminal of the sequence shown in SEQ ID No:4 at any amino acid site within the α5 region thereof;

the N-terminal fragment of the HPV type 11 L1 protein has 98%, 98.5%, 99%, 99.5%, 99% or 100% identity with a fragment obtained by truncating the C-terminal of the sequence shown in SEQ ID No:5 at any amino acid site within the α5 region thereof;
the N-terminal fragment of the HPV type 16 L1 protein has 98%, 98.5%, 99%, 99.5%, 99% or 100% identity with a fragment obtained by truncating the C-terminal of the sequence shown in SEQ ID No:6 at any amino acid site within the α5 region thereof;
the N-terminal fragment of the HPV type 18 L1 protein has 98%, 98.5%, 99%, 99.5%, 99% or 100% identity with a fragment obtained by truncating the C-terminal of the sequence shown in SEQ ID No:7 at any amino acid site within the α5 region thereof;
the N-terminal fragment of the HPV type 31 L1 protein has 98%, 98.5%, 99%, 99.5%, 99% or 100% identity with a fragment obtained by truncating the C-terminal of the sequence shown in SEQ ID No:8 at any amino acid site within the α5 region thereof;
the N-terminal fragment of the HPV type 35 L1 protein has 98%, 98.5%, 99%, 99.5%, 99% or 100% identity with a fragment obtained by truncating the C-terminal of the sequence shown in SEQ ID No:9 at any amino acid site within the α5 region thereof;

the N-terminal fragment of the HPV type 39 L1 protein has 98%, 98.5%, 99%, 99.5%, 99% or 100% identity with a fragment obtained by truncating the C-terminal of the sequence shown in SEQ ID No: 10 at any amino acid site within the α5 region thereof;

the N-terminal fragment of the HPV type 45 L1 protein has 98%, 98.5%, 99%, 99.5%, 99% or 100% identity with a fragment obtained by truncating the C-terminal of the sequence shown in SEQ ID No:11 at any amino acid site within the α5 region thereof;

the N-terminal fragment of the HPV type 51 L1 protein has 98%, 98.5%, 99%, 99.5%, 99% or 100% identity with a fragment obtained by truncating the C-terminal of the sequence shown in SEQ ID No:12 at any amino acid site within the α5 region thereof;

the N-terminal fragment of the HPV type 52 L1 protein has 98%, 98.5%, 99%, 99.5%, 99% or 100% identity with a fragment obtained by truncating the C-terminal of the sequence shown in SEQ ID No:13 at any amino acid site within the α5 region thereof;

the N-terminal fragment of the HPV type 56 L1 protein has 98%, 98.5%, 99%, 99.5%, 99% or 100% identity with a fragment obtained by truncating the C-terminal of the sequence shown in SEQ ID No:14 at any amino acid site within the α5 region thereof; and

the N-terminal fragment of the HPV type 58 L1 protein has 98%, 98.5%, 99%, 99.5%, 99% or 100% identity with a fragment obtained by truncating the C-terminal of the sequence shown in SEQ ID No:15 at any amino acid site within the α5 region thereof.

In one embodiment, the C-terminal of the N-terminal fragment is connected directly to the N-terminal of the C-terminal fragment or by a linker.

In one embodiment, when the C-terminus of said N-terminal fragment is connected to the N-terminus of said C-terminal fragment, the continuous amino acid sequence RKFL is present within a range of plus or minus 4 amino acid positions of the splicing site,

Preferably, the continuous amino acid sequence LGRKFL is present within a range of plus or minus 6 amino acid positions of the splicing site.

In one embodiment, the chimeric HPV type 6, 11, 16, 18, 31, 35, 39, 45, 51, 52, 56, and 58 chimeric HPV L1 proteins have 98%, 98.5%, 99%, 99.5% or 100% identity to SEQ ID No:16, SEQ ID No:17, SEQ ID No:18, SEQ ID No:19, SEQ ID No:20, SEQ ID No:21, SEQ ID No:22, SEQ ID No:23, SEQ ID No:24, SEQ ID No:25, SEQ ID No:26 and SEQ ID No: 27, respectively; and the HPV type 33 L1 protein and the HPV type 59 L1 protein have 98%, 98.5%, 99%, 99.5% or 100% identity to SEQ ID No:28 and SEQ ID No:29, respectively.

In one embodiment, the formulation comprises HPV types 6, 11, 16, 18, 31, 35, 39, 45, 51, 52, 56 and 58 chimeric HPV L1 protein having the amino acid sequences shown in SEQ ID No:16, SEQ ID No:17, SEQ ID No:18, SEQ ID No:19, SEQ ID No:20, SEQ ID No:21, SEQ ID No:22, SEQ ID No:23, SEQ ID No:24, SEQ ID No:25, SEQ ID No:26 and SEQ ID No: 27, respectively; and

HPV type 33 L1 protein and HPV type 59 L1 protein having the amino acid sequences shown in SEQ ID No:28 and SEQ ID No: 29, respectively.

In one aspect, the present invention provides a method of preventing an HPV-related disease or infection comprising administering to a subject a stable formulation of the multivalent human papillomavirus virus-like particle vaccine formulation. The prevention may be considered as a treatment and the two terms are used interchangeably. In one embodiment, the subject is human.

In one aspect, the formulation is stable at 2 to 8 °C for at least 24 month and at 25 °C for at least 16 weeks.

In one aspect, the present invention provides the use of the human papillomavirus virus-like particle vaccine formulation in the preparation of a vaccine for the prevention of HPV-related diseases or infections.

## BRIEF DESCRIPTION OF THE DRAWINGS

Figure 1 shows the results of the adsorption degree test of each formulation sample in Example 1.

Figure 2 shows the analysis result of antigen content of each formulation sample in Example 1. T0:37 °C, week 0; 37 °C_1W: 37 °C, week 1; 37 °C _ 2 W: 37 °C, week 2; 37 °C 4 W: 37 °C week 4.

Figure 3 shows the results of the adsorption degree test of each formulation sample in Example 2.

Figure 4 shows the analysis results of antigen content of each formulation sample in Example 2. T0:37 °C, week 0; 37 °C _1W: 37 °C, week 1; 37 °C _ 2 W: 37 °C, week 2; 37 °C 4 W: 37 °C week 4.

Figure 5 shows the results of the adsorption degree test of each formulation sample in Example 3.

Figure 6 shows the analysis results of antigen content of each formulation sample in Example 3. T0:37 °C, week 0; 37 °C _1W: 37 °C, week 1; 37 °C _ 2 W: 37 °C, week 2; 37 °C 4 W: 37 °C week 4.

Figure 7 shows the results of the adsorption degree test of each formulation sample in Example 4.

Figure 8 shows the analysis results of antigen content of each formulation sample in Example 4. T0:37 °C, week 0; 37 °C _1W: 37 °C, week 1; 37 °C _ 2 W: 37 °C, week 2; 37 °C 4 W: 37 °C week 4.

## DETAILED DESCRIPTION

The invention provides a stable formulation of a human papillomavirus vaccine, solves the problem of antibody stability in the processes of storage and transportation, and ensures that the pre-dose antigen still has the immunogenicity and safety satisfying the prevention purpose

The term "formulation" refers to a composition that maintains the biological activity of the active component in an effective manner and does not contain other components that are unacceptably toxic to the subject. Such formulations are sterile. The term "sterile" refers to the absence of live bacteria or the absence or substantial absence of all live microorganisms and their spores.

As used herein, a "stable" formulation refers to a formulation in which the active ingredient substantially retains its physical and/or chemical stability and/or biological activity after storage. Preferably, the formulation substantially retains its physical and chemical stability as well as its biological activity after storage.

The terms "patient" or "subject" are used interchangeably and refer to any mammal suffering from a condition or disease in accordance with the present invention. Preferably, human.

As used herein, "physiologically acceptable" means a concentration or ionic strength of a buffer, excipient, or salt makes the formulation biologically compatible with the immunized target host, e.g., human.

The stable formulation of the present invention comprises human papillomavirus virus-like particles, a buffer, an osmotic pressure regulator, and an aluminum adjuvant.

The terms "comprising" and "containing" mean that additional components may be included in addition to the components mentioned.

As use herein and in that appended claim, the singular forms "a", "an", "the" and "said" include plural referents unless the context clearly dictates otherwise.

As used herein, "buffer" refers to a buffer solution that resists pH change by the action of its conjugate acid-base pair. In one embodiment of the present invention, histidine buffer is used and the pH of the formulation solution is preferably about 5.9 to 6.5, more preferably 6.2.

As used herein, "surfactant" refers to a surface active agent, and in one embodiment, the surfactant herein is polysorbate 80.

The term "osmotic pressure regulator" means a pharmaceutically acceptable osmotic pressure regulator. Suitable osmotic regulators include, but are not limited to salts, and in one embodiment of the present invention are sodium chloride (NaCl) with a concentration of about 150mM to 320mM.

The term "adjuvant" refers to a compound or mixture that enhances an immune response. In particular, the vaccine may comprise an adjuvant. Adjuvants used in the present invention are selected from one or more of aluminum hydroxyphosphate ($AlPO_4$), amorphous aluminum hydroxyphosphate sulfate (AAHS), or aluminum hydroxide ($Al(OH)_3$), preferably aluminum hydroxyphosphate ($AlPO_4$).

The "stability" of protein after storage at a selected temperature for a selected period of time may be assessed qualitatively and/or quantitatively in a number of different ways. In the embodiment of the invention, Enzyme-linked immunosorbent assay (ELISA) was used to measure the content of active antigen binding to the recombinant human papillomavirus neutralizing antibody, and the ratio of the active antigen content at each time point to T0 was used to compare the stability of corresponding formulation; Enzyme-linked immunosorbent assay (ELISA) was used to measure the content of the antigen not adsorbed on aluminum phosphate adjuvant with the aid of centrifugation and then to calculate the absorption degree; Determining the $EC_{50}$ of the human papillomavirus vaccine formulation and the positive control to the recombinant human papillomavirus neutralizing antibody respectively , calculating the $EC_{50}$ ratio of the vaccine formulation to the positive control thereby determining the *in vitro* relative potency of the vaccine.

The term "immunogenicity" refers to the ability of a substance, such as a protein or polypeptide, to stimulate an immune response, i.e., to stimulate the production of antibodies, particularly responses that generates humoral or stimulated cell-mediated responses.

The term "HPV" or "HPV virus" refers to a papillomavirus of the family *Papillomaviridae,* which is an uncoated DNA virus having a double-stranded closed-loop DNA genome of about 8kb in size and which can generally be divided into three regions: ① the early region (E) comprising six open reading frames encoding nonstructural proteins related to E1, E2, E4-E7 virus replication, transcription and transformation, as well as E3 and E8 open reading frames; ② the late region (L) comprises a reading frame encoding the major capsid protein L1 and the minor capsid protein L2; ③ long regulatory region (LCR) does not encode any protein, but it has the origin of replication and multiple transcription factor binding sites.

The terms "HPV L1 protein" and "HPV L2 protein" refer to proteins encoded by the late region (L) of the HPV gene and synthesized in the middle and late period of the HPV infection cycle. L1 protein is the major capsid protein and has a molecular weight of 55-60kDa. L2 protein is the minor capsid protein. Seventy-two L1 pentamers form the shell of icosahedral HPV virus particles, wrapping the closed-loop double-stranded DNA micro-chromosome. L2 protein is located inner lining of the L1 protein.

The term "virus-like particles" is a hollow particle containing one or more structural proteins of a virus without viral nucleic acid.

The term "concentration of papillomavirus virus-like particle of any single type" refers to the content of papillomavirus virus-like particle of any single type in the formulation, and the term "total concentration of papillomavirus virus-like particles of all types" is the sum of the concentrations of papillomavirus virus-like particle of each single type included in the formulation.

In one embodiment of the invention, the human papillomavirus multivalent immunogenic composition described in patent application PCT/CN2020/102601 filed on July 17, 2020 is adopted, and PCT/CN2020/102601 is incorporated by reference into the present specification and claims.

In a particularly preferred embodiment of the invention, the formulation comprises 0.74mg/mL of papillomavirus virus-like particles, 1.0mg/mL of aluminum phosphate adjuvant, 18mM histidine buffer, 320mM sodium chloride, and a pH of 6.2. Wherein the vaccine comprises polysorbate 80 at a concentration of not more than 0.3mg/mL due to process residues during preparation. The formulation has good stability, and can be stably stored at 2 to 8 °C for at least 24 months and at 25 °C for at least 16 weeks.

The formulations of the present invention may be provided in liquid form or may be provided in lyophilized form. The lyophilized formulation may be reconstituted prior to administration.

## EXAMPLE

The present invention will be more fully understood by reference to the following examples. However, they should not be construed as limiting the scope of the invention. All documents, patents and patent applications are incorporated herein by reference.

In the examples below, the preparation, characterization and performance identification of various types of papillomavirus virus-like particles used are described in patent application PCT/CN2020/102601, filed on July 17, 2020.

In the following examples, the detection method used was as follows:

1) Analysis of antigen content (Enzyme-linked Immunosorbent Assay (ELISA)

The positive control (human papillomavirus virus-like particle standard, source: SinoCellTech Ltd., chimeric HPV type 6, 16, 18, 31, 35, 30, 45, 51, 52, and 56 chimeric HPV L1 protein and HPV type 33 and HPV type 59 L1 protein, corresponding to the amino acid sequence of SEQ ID NO:16-29 respectively, the same as below) and the analyte were completely dissolved using the desorption buffer, serving as the positive control and the analyte to be detected.

The recombinant human papillomavirus neutralizing antibody (source: Sino Biological, Inc., the same as below) was combined with a solid-phase carrier to form a solid-phase antibody. Diluting the positive control and the analyte to be detected with the sample diluent, and then combining with the solid phase antibody to form a solid phase antigen antibody complex; enzyme-labeled antibody was then added and the substrate was added for developing, and the color product was read at a wavelength of 450nm. Performing linear regression on the concentration of a series of positive controls and the corresponding absorbance, substituting the absorbance value of the analyte into a linear regression equation, and obtaining the antigen content of the sample to be detected (M. Shank-Retzlaff, F. Wang, T. Morley et al. Correlation between Mouse Potency and In Vitro Relative Potency for Human Papillomavirus Type 16 Virus-Like Particles and Gardasil Vaccine Samples. Human Vaccines, 1:5, 191-197).

2) Adsorption degree analysis (enzyme-linked immunosorbent assay (ELISA)

The recombinant human papillomavirus neutralizing antibody is combined with a solid phase carrier to form a solid phase antibody. Centrifuging the sample to be detected, and taking the supernatant as an analyte. Diluting the positive control and the analyte accordingly with the sample diluent, and then combining with the solid phase antibody to form a solid phase antigen antibody complex; enzyme-labeled antibody was then added and the substrate was added for developing, and the color product was read at a wavelength of 450nm. Performing linear regression on the concentration of a series of positive controls and the corresponding absorbance, substituting the absorbance value measured with the analyte into a linear regression equation to obtain the antigen concentration of the supernatant, and calculating the adsorption degree of the sample to be detected by the following equation (Michael J. Caulfield, Li Shi, Su Wang et al. Effect of Alternative Aluminum Adjuvants on the Absorption and Immunogenicity of HPV16 L1 VLPs in Mice. Human Vaccines 3:4, 139-146).

$$\text{Adsorption degree (\%)} = (1\text{- antigen concentration in supernatant/antigen concentration of sample to be detected)\%}.$$

3) Determination of *In Vitro* Relative Potency, IVRP (IVRP)

The positive control (14 valent human papillomavirus vaccine (types 6, 11, 16, 18, 31, 33, 35, 39, 45, 51, 52, 56, 58 and 59) control product obtained from SinoCellTech Ltd, with the sequence same as the protein sequence in the test sample) and the analyte were completely dissolved using desorption buffer, and used as the positive control and the analyte to be detected. The recombinant human papillomavirus neutralizing antibody was diluted to a final concentration of $2\mu g/mL$, added to 96-well plates at $100\mu L/$ well, the plates were tapped to mix the samples, and the samples were coated overnight at 4 °C; the plates were washed with washing solution at a dose of $200\mu L/$ well once and the ELISA plate was dried. Then the ELISA plate was blocked with blocking solution at $300\mu L/$ well for one hour at room temperature. The samples were washed twice with washing solution at $300\mu L/$ well, and $100\mu L$ treated blank control (buffer solution corresponding to analyte), positive control and analyte to be detected were added into each well and incubated for 1 hour at room temperature. After the plates were washed three times with washing solution at $200\mu L/$ well, diluted enzyme-labeled recombinant human papillomavirus neutralizing antibody was added at $100\mu L/$ well. After incubated at room temperature for 1 h, the plates were washed with washing solution at $200\mu L/$ well for three times, and the developing solution was added at $200\mu L/$ well and placed at room temperature for $20\pm5$min. The reaction was terminated by adding a stop solution at $50\mu L/$well; the absorbance at 450nm was detected by a microplate reader. Processing by using a computer program Origin or a four-parameter fitting method, taking the concentration of the positive control or the analyte as an abscissa and the average absorbance as an ordinate to obtain the $EC_{50}$ of the analyte and the positive control, and dividing the $EC_{50}$ of the analyte by the $EC_{50}$ of the positive control to obtain the *in vitro* relative potency of the analyte(M. Shank-Retzlaff, F. Wang, T. Morley et al. Correlation between Mouse Potency and In Vitro Relative Potency for Human Papillomavirus Type 16 Virus-Like Particles and Gardasil Vaccine Samples. Human Vaccines, 1:5, 191-197).

Example 1: Screening study of surfactant concentration

The composition of the papillomavirus virus-like particle vaccine formulation of this example is shown in the following table:

Table 1 Formulations of different surfactant concentrations

| Composition number | Papillomavirus virus-like particle | Aluminum phosphate adjuvant | Histidine buffer | NaCl | Polysorbate 80 | pH |
|---|---|---|---|---|---|---|
| F1 | 0.74mg/mL | 1.0mg/mL | 18mM | 320mM | 0.00wt% | 6.5 |
| F2 | 0.74mg/mL | 1.0mg/mL | 18mM | 320mM | 0.02wt% | 6.5 |

Preparation method of papillomavirus virus-like particle vaccine formulation:
Took a certain amount of HPV 18 virus-like particles appropriate to the formulation, and then mixed it with aluminum phosphate adjuvant, and adsorbed it overnight at 4 °C, so that the pH, the corresponding concentrations of papillomavirus virus-like particles, aluminum phosphate adjuvant, histidine, sodium chloride, and polysorbate 80 in the papillomavirus virus-like particle vaccine formulation respective met the requirements of Table 1, dispensed it in aliquot. Marked it with the corresponding numbers, placed the samples in a 37 °C incubator, and took it out for analysis of adsorption degree and antigen content at week 0, and for antigen content analysis at weeks 1, 2, and 4.

Analytical test method:

Adsorption degree analysis: the detection principle is that antigens not adsorbed onto aluminum phosphate adjuvant are obtained by centrifugation and the content is analyzed to calculate the adsorption degree.
Antigen content analysis: the detection principle is that the active antigen content capable of binding to the recombinant human papillomavirus neutralizing antibody is measured by ELISA, and the stability of each formulation is compared by comparing the ratio of the active antigen content at each time point with that of T0. The higher the ratio, the higher the active antigen content in the formulation and the better the activity maintained.

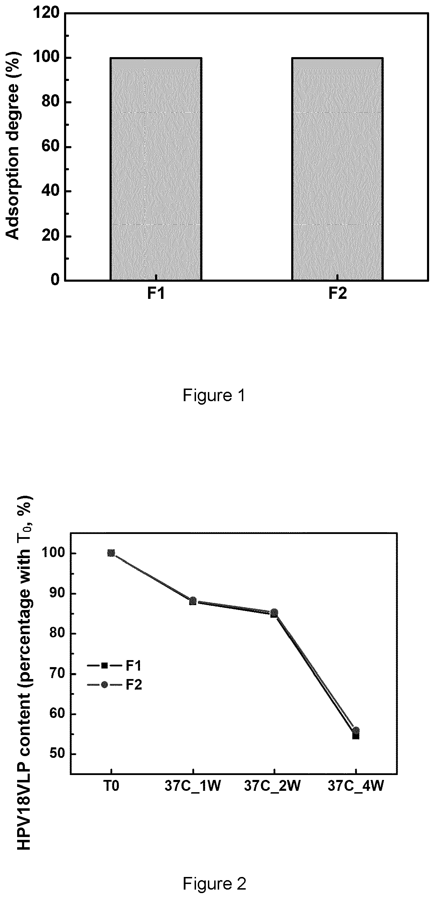

The test results are shown in tables 2-3 and figures 1-2.

Table 2 Adsorption degree of HPV18 virus-like particle formulation

| Num | %HPV18 VLPs binding to aluminum adjuvant |
| --- | --- |
| F1 | 99.8 |
| F2 | 99.9 |

Table 3 Antigen content of HPV18 virus-like particle formulation

| Time point | HPV18VLP content (percentage with $T_0$, %) | |
| --- | --- | --- |
| | F1 | F2 |
| $T_0$ | 100 | 100 |
| 37°C_1W | 87.9 | 88.2 |
| 37 °C_2W | 84.8 | 85.3 |
| 37 °C_4W | 54.5 | 55.9 |

The test results showed that the adsorption degrees of both two papillomavirus virus-like particle vaccine formulations were above 99%, and there was no significant difference in the changes of antigen content between F1 and F2, that is, the stability of F1 and F2 was comparable.

Example 2: Screening study of pH

The composition of the papillomavirus virus-like particle vaccine formulation of this example is shown in the following table:

Table 4 Formulations with different pH

| Composition number | Papillomavirus virus-like particle | Aluminum phosphate adjuvant | Histidine buffer | NaCl | Polysorbate 80 | pH |
| --- | --- | --- | --- | --- | --- | --- |
| F1 | 0.74mg/mL | 1.0mg/mL | 18mM | 320mM | 0.00wt% | 5.9 |
| F2 | 0.74mg/mL | 1.0mg/mL | 18mM | 320mM | 0.00wt% | 6.2 |
| F3 | 0.74mg/mL | 1.0mg/mL | 18mM | 320mM | 0.00wt% | 6.5 |

Preparation method of papillomavirus virus-like particle vaccine formulation:

Took a certain amount of HPV 18 virus-like particles appropriate to the formulation, and then mixed it with aluminum phosphate adjuvant, and adsorbed it overnight at 4 °C, so that the pH, the corresponding concentrations of papillomavirus virus-like particles, aluminum phosphate adjuvant, histidine, sodium chloride, and polysorbate 80 in the papillomavirus virus-like particle vaccine formulation respective met the requirements of Table 4, dispensed it in aliquot. Marked it with the corresponding numbers, placed the samples in a 37 °C incubator, and took it out for analysis of adsorption degree and antigen content at week 0, and for antigen content analysis at weeks 1, 2, and 4.

Analytical test method:

Adsorption degree analysis: the detection principle is that antigens not adsorbed onto aluminum phosphate adjuvant are obtained by centrifugation and the content is analyzed to calculate the adsorption degree.

Antigen content analysis: the detection principle is that the active antigen content capable of binding to the recombinant human papillomavirus neutralizing antibody is measured by ELISA, and the stability of each formulation is compared by comparing the ratio of the active antigen content at each time point with that of T0. The higher the ratio, the higher the active antigen content in the formulation and the better the activity maintained.

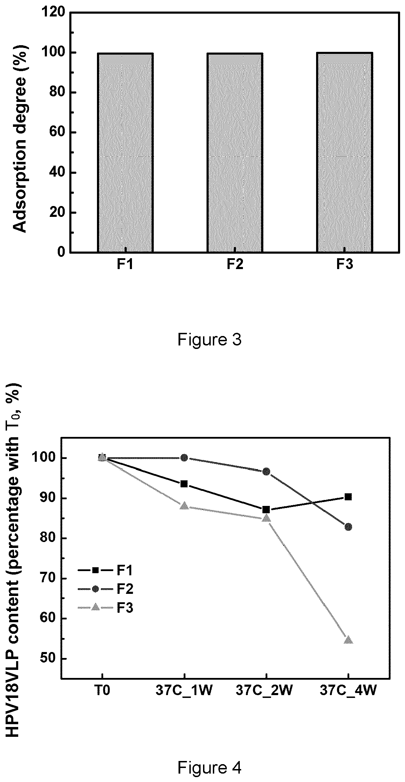

The test results are shown in tables 5-6 and figures 3-4.

Table 5 Adsorption degree of HPV18 virus-like particle formulation

| Num | %HPV18 VLPs binding to aluminum adjuvant |
|---|---|
| F1 | 99.4 |
| F2 | 99.4 |
| F3 | 99.8 |

Table 6 Antigen content of HPV18 virus-like particle formulation

| Time point | HPV18VLP content (percentage with $T_0$, %) | | |
|---|---|---|---|
| | F1 | F2 | F3 |
| $T_0$ | 100 | 100 | 100 |
| 37°C_1W | 93.5 | 100 | 87.9 |
| 37 °C_2W | 87.1 | 96.6 | 84.8 |
| 37 °C_4W | 90.3 | 82.8 | 54.5 |

The test results showed that the adsorption degrees of the three papillomavirus virus-like particle vaccine formulations were above 99%, and the antigen content change trends of the papillomavirus virus-like particle vaccines in F1 and F2 were better than that of the F3 formulation, that is, the stability of F1 and F2 was better than that of F3.

Example 3: Screening study of osmotic pressure regulator concentration

The composition of the papillomavirus virus-like particle vaccine formulation of this example is shown in the following table:

Table 7 Formulations of different osmotic pressure regulator concentrations

| Composition number | Papillomavirus virus-like particle | Aluminum phosphate adjuvant | Histidine buffer | NaCl | Polysorbate 80 | pH |
|---|---|---|---|---|---|---|
| F1 | 0.74mg/mL | 1.0mg/mL | 18mM | 320mM | 0.00wt% | 6.2 |
| F2 | 0.74mg/mL | 1.0mg/mL | 18mM | 150mM | 0.00wt% | 6.2 |

Preparation method of papillomavirus virus-like particle vaccine formulation:

Took a certain amount of HPV 18 virus-like particles appropriate to the formulation, and then mixed it with aluminum phosphate adjuvant, and adsorbed it overnight at 4 °C, so that the pH, the corresponding concentrations of papillomavirus virus-like particles, aluminum phosphate adjuvant, histidine, sodium chloride, and polysorbate 80 in the papillomavirus virus-like particle vaccine formulation respective met the requirements of Table 7, dispensed it in aliquot. Marked it with the corresponding numbers, placed the samples in a 37 °C incubator, and took it out for analysis of adsorption degree and antigen content at week 0, and for antigen content analysis at weeks 1, 2, and 4.

Analytical test method:

Adsorption degree analysis: the detection principle is that antigens not adsorbed onto aluminum phosphate adjuvant are obtained by centrifugation and the content is analyzed to calculate the adsorption degree.

Antigen content analysis: the detection principle is that the active antigen content capable of binding to the recombinant human papillomavirus neutralizing antibody is measured by ELISA, and the stability of each formulation is compared by comparing the ratio of the active antigen content at each time point with that of T0. The higher the ratio, the higher the active antigen content in the formulation and the better the activity maintained.

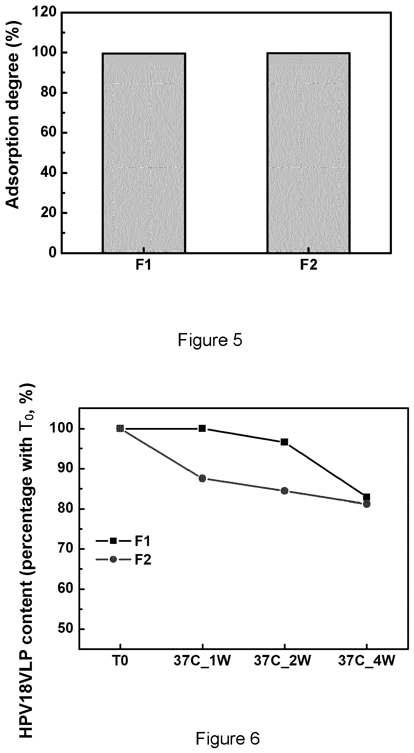

The test results are shown in tables 8-9 and figures 5-6.

Table 8 Adsorption degree of HPV18 virus-like particle formulation

| Num | %HPV18 VLPs binding to aluminum adjuvant |
|---|---|
| F1 | 99.4 |
| F2 | 99.6 |

Table 9 Antigen content of HPV18 virus-like particle formulation

| Time point | HPV18VLP content (percentage with $T_0$, %) | |
|---|---|---|
| | F1 | F2 |
| $T_0$ | 100 | 100 |
| 37°C_1W | 87.9 | 88.2 |
| 37 °C_2W | 84.8 | 85.3 |
| 37 °C_4W | 54.5 | 55.9 |

The test results showed that the adsorption degrees of the two papillomavirus virus-like particle vaccine formulations were above 99%. There was no significant difference in the active antigen content s changes of F1 and F2 formulations at 4 weeks under 37 °C, that is, the stability of F1 and F2 was comparable.

Example 4: screening study of buffer concentration

The composition of the papillomavirus virus-like particle vaccine formulation of this example is shown in the following table:

Table 10 Formulations of different buffer concentrations

| Composition number | Papillomavirus virus-like particle | Aluminum phosphate adjuvant | Histidine buffer | NaCl | Polysorbate 80 | pH |
|---|---|---|---|---|---|---|
| F1 | 0.74mg/mL | 1.0mg/mL | 10mM | 320mM | 0.00wt% | 6.2 |
| F2 | 0.74mg/mL | 1.0mg/mL | 18mM | 320mM | 0.00wt% | 6.2 |
| F3 | 0.74mg/mL | 1.0mg/mL | 26mM | 320mM | 0.00wt% | 6.2 |

Preparation method of papillomavirus virus-like particle vaccine formulation;

Took a certain amount of HPV 18 virus-like particles appropriate to the formulation, and then mixed it with aluminum phosphate adjuvant, and adsorbed it overnight at 4 °C, so that the pH, the corresponding concentrations of papillomavirus virus-like particles, aluminum phosphate adjuvant, histidine, sodium chloride, and polysorbate 80 in the papillomavirus virus-like particle vaccine formulation respective met the requirements of Table 10, dispensed it in aliquot. Marked it with the corresponding numbers, placed the samples in a 37 °C incubator, and took it out for analysis of adsorption degree and antigen content at week 0, and for antigen content analysis at weeks 1, 2, and 4.

Analytical test method:

Adsorption degree analysis: the detection principle is that antigens not adsorbed onto aluminum phosphate adjuvant are obtained by centrifugation and the content is analyzed to calculate the adsorption degree.

Antigen content analysis: the detection principle is that the active antigen content capable of binding to the recombinant human papillomavirus neutralizing antibody is measured by ELISA, and the stability of each formulation is compared by comparing the ratio of the active antigen content at each time point with that of T0. The higher the ratio, the higher the active antigen content in the formulation and the better the activity maintained.

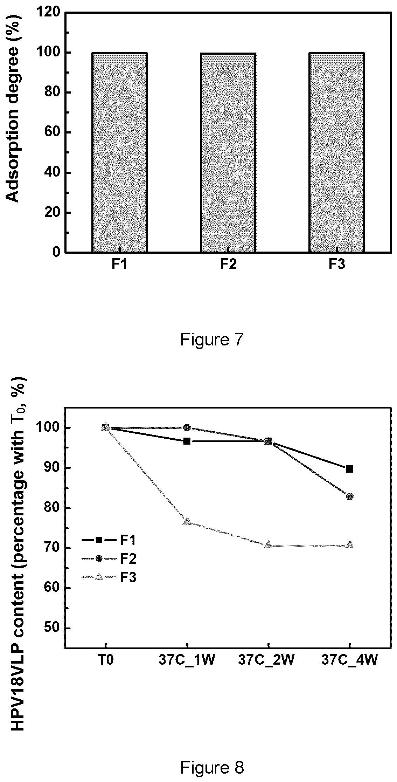

The test results are shown in tables 11-12 and figures 7-8.

Table 11 Adsorption degree of HPV18 virus-like particle formulation

| Num | %HPV18 VLPs binding to aluminum .adjuvant |
| --- | --- |
| F1 | 99.6 |
| F2 | 99.4 |
| F3 | 99.6 |

Table 12 antigen content of HPV18 virus-like particle formulation

| Time point | HPV18VLP content (percentage with $T_0$, %) | | |
| --- | --- | --- | --- |
| | F1 | F2 | F3 |
| $T_0$ | 100 | 100 | 100 |
| 37°C_1W | 96.6 | 100 | 76.5 |
| 37 °C_2W | 96.6 | 96.6 | 70.6 |
| 37 °C_4W | 89.7 | 82.8 | 70.6 |

The test results showed that the adsorption degrees of the three papillomavirus virus-like particle vaccine formulations were above 99%, and the antigen content change trends of the papillomavirus virus-like particle vaccines in F1 and F2 were better than those of the F3 formulation, that is, the stability of F1 and F2 was better than that of F3.

Example 5: Composition confirmation study of each single type (type 6, 11, 16, 18, 31, 33, 35, 39, 45, 51, 52, 56, 58, 59) papillomavirus virus-like particles vaccine formulation

Each single type (type 6, 11, 16, 18, 31, 33, 35, 39, 45, 51, 52, 56, 58 and 59) of papillomavirus virus-like particle vaccine formulations (formulation: 0.74mg/mL papillomavirus virus-like particle +1.0mg/mL aluminum phosphate adjuvant +18mM histidine buffer +320mM sodium chloride at pH 6.2) was subjected to stability monitoring at 2-8 °C, respectively. Wherein the vaccine contained polysorbate 80 at a concentration of not more than 0.3mg/mL due to process residues in preparation. The monitoring time points were 3m(3 months), 6m(6 months), 9m(9 months) and 12m(12 months), and the adsorption degree and *in vitro* relative potency were monitored.

Analytical test method:

Adsorption degree analysis: the detection principle is that antigens not adsorbed onto aluminum phosphate adjuvant are obtained by centrifugation and the content is analyzed to calculate the adsorption degree.

*In vitro* relative potency: the detection principle of this method is to detect the EC50 of the analyte and the positive control with the recombinant human papillomavirus neutralization antibody respectively, then to calculate the percentage ration of $EC_{50}$ thereof. The higher the value, the higher the *in vitro* relative efficacy and the better quality of the test sample.

The experimental results are shown in Table 13.

Experimental results show that each single type of papillomavirus virus-like particle vaccine formulations disclosed by the invention has good stability and can be stably stored for at least 12 months under the condition of 2-8 °C .

Table 13 Stability data of all 14 single types (6, 11, 16, 18, 31, 33, 35, 39, 45, 51, 52, 56, 58, and 59) Papillomavirus-like particles adjuvant adsorption liquid composition

| Papillomavirus virus-like particle type | Adsorption degree (%) | | | | | *In vitro* relative potency (%) | | | | |
| --- | --- | --- | --- | --- | --- | --- | --- | --- | --- | --- |
| | T0 | 4°C-3m | 4°C-6m | 4°C-9m | 4°C-12m | T0 | 4°C-3m | 4°C-6m | 4°C-9m | 4°C-12m |
| 6 | 98 | 99 | 99 | 100 | >99 | 101 | 117 | 99 | 116 | 113 |
| 11 | 99 | 100 | 100 | >99 | >99 | 96 | 113 | 108 | 143 | 113 |
| 16 | >99 | >99 | >99 | >99 | >99 | 92 | 116 | 100 | 130 | 97 |
| 18 | >99 | >99 | >99 | >99 | >99 | 86 | 110 | 106 | 97 | 109 |
| 31 | >99 | >99 | >99 | >99 | >99 | 109 | 108 | 98 | 113 | 92 |

(continued)

| Papillomavirus virus-like particle type | Adsorption degree (%) | | | | | In vitro relative potency (%) | | | | |
|---|---|---|---|---|---|---|---|---|---|---|
| | T0 | 4°C-3m | 4°C-6m | 4°C-9m | 4°C-12m | T0 | 4°C-3m | 4°C-6m | 4°C-9m | 4°C-12m |
| 33 | >99 | >99 | >99 | >99 | >99 | 96 | 113 | 107 | 108 | 103 |
| 35 | >99 | >99 | >99 | >99 | >99 | 85 | 86 | 91 | 88 | 97 |
| 39 | >99 | >99 | >99 | >99 | >99 | 101 | 110 | 109 | 105 | 102 |
| 45 | 99 | >99 | >99 | >99 | >99 | 88 | 119 | 115 | 107 | 120 |
| 51 | >99 | >99 | >99 | >99 | >99 | 94 | 118 | 98 | 110 | 117 |
| 52 | >99 | >99 | >99 | >99 | >99 | 90 | 120 | 104 | 103 | 106 |
| 56 | >99 | >99 | >99 | >99 | >99 | 90 | 108 | 99 | 92 | 101 |
| 58 | >99 | >99 | >99 | >99 | >99 | 97 | 98 | 106 | 113 | 108 |
| 59 | >99 | >99 | >99 | >99 | >99 | 96 | 104 | 106 | 114 | 115 |

Example 6 Formulation preparation and composition confirmation of 14 valent human papillomavirus vaccine (type 6, 11, 16, 18, 31, 33, 35, 39, 45, 51, 52, 56, 58, 59)

Preparation method of 14 valent human papillomavirus vaccine (type 6, 11, 16, 18, 31, 33, 35, 39, 45, 51, 52, 56, 58, 59):

Each single type (type 6,11,16,18, 31, 33, 35, 39, 45, 51, 52, 56, 58 and 59) of papillomavirus virus-like particle vaccine formulation (Composition: 0.74mg/mL papillomavirus virus-like particles +1.0mg/mL aluminum phosphate adjuvant +18mM histidine buffer +320mM sodium chloride, pH value: 6.2) was respectively taken and mixed in a certain volume ratio (type 6, 11, 16, 18, 31, 33, 35, 39, 45, 51, 52, 56, 58, 59 =1.5:2:3:2: 1: 1: 1: 1: 1: 1: 1: 1: 1: 1) to obtain a 14 valent human papillomavirus vaccine (type 6, 11, 16, 18, 31, 33, 35, 39, 45, 51, 52, 56, 58, 59) semi-finished product, corresponding to the concentration of 0.06mg/mL, 0.08mg/mL, 0.12mg/mL, 0.08mg/mL, 0.04mg/mL, 0.04mg/mL, 0.04mg/mL, 0.04mg/mL, 0.04mg/mL, 0.04mg/mL, 0.04mg/mL, 0.04mg/mL, 0.04mg/mL, 0.04mg/mL of each type of virus-like particle, respectively; then filled into Penicillin vials which were then corked, capped, and labeled to prepare recombinant 14 valent human papillomavirus vaccine (type 6, 11, 16, 18, 31, 33, 35, 39, 45, 51, 52, 56, 58 and 59), wherein the vaccine comprised polysorbate 80 in a concentration of not more than 0.3mg/mL due to process residues in the preparation process. The stability monitoring was then conducted at 2-8 °C (time points are 3, 6, 9, 12, 18, and 24 months) and 25±2 °C (time points are 2 weeks, 4 weeks, 8 weeks, and 16 weeks), and the in vitro relative potency and adsorption degree were monitored.

Analytical test method:

Adsorption degree analysis: the detection principle is that antigens not adsorbed onto aluminum phosphate adjuvant are obtained by centrifugation and the content is analyzed to calculate the adsorption degree.

Antigen content analysis: the detection principle is that the active antigen content capable of binding to the recombinant human papillomavirus neutralizing antibody is measured by ELISA, and the stability of each formulation is compared by comparing the ratio of the active antigen content at each time point with that of T0. The higher the ratio, the higher the active antigen content in the formulation and the better the activity maintained.

The experimental results are shown in tables 14-17.

Table 14 *In vitro* relative potency (25 °C ) of recombinant 14 valent human papillomavirus vaccine (type 6, 11, 16, 18, 31, 33, 35, 39, 45, 51, 52, 56, 58, 59)

| Lot \ Time \ Type | *In vitro* relative potency(%) | | | | | | | | | | | | | |
|---|---|---|---|---|---|---|---|---|---|---|---|---|---|---|
| | **6** | **11** | **16** | **18** | **31** | **33** | **35** | **39** | **45** | **51** | **52** | **56** | **58** | **59** |
| **Lot1** 0 weeks | 95 | 97 | 95 | 107 | 97 | 95 | 107 | 95 | 97 | 103 | 106 | 93 | 97 | 89 |
| 2 weeks | 90 | 96 | 89 | 85 | 96 | 97 | 91 | 83 | 86 | 88 | 84 | 92 | 87 | 90 |
| 4 weeks | 91 | 93 | 87 | 79 | 85 | 101 | 103 | 101 | 99 | 94 | 106 | 95 | 94 | 92 |
| 8 weeks | 92 | 92 | 89 | 88 | 88 | 87 | 87 | 105 | 81 | 92 | 88 | 93 | 88 | 91 |
| 16 weeks | 89 | 85 | 95 | 82 | 80 | 87 | 85 | 96 | 84 | 94 | 89 | 88 | 90 | 85 |
| **Lot2** 0 weeks | 103 | 95 | 93 | 114 | 113 | 93 | 118 | 113 | 94 | 88 | 107 | 101 | 113 | 99 |
| 2 weeks | 86 | 92 | 84 | 92 | 87 | 88 | 87 | 96 | 84 | 88 | 78 | 93 | 97 | 87 |
| 4 weeks | 94 | 89 | 75 | 84 | 82 | 90 | 100 | 92 | 93 | 94 | 96 | 92 | 101 | 96 |
| 8 weeks | 97 | 90 | 86 | 93 | 79 | 84 | 84 | 104 | 76 | 100 | 80 | 90 | 93 | 96 |
| 16 weeks | 86 | 74 | 90 | 79 | 69 | 87 | 80 | 96 | 78 | 101 | 73 | 68 | 80 | 82 |
| **Lot3** 0 weeks | 90 | 97 | 98 | 105 | 118 | 91 | 115 | 106 | 95 | 93 | 102 | 92 | 118 | 98 |
| 2 weeks | 76 | 94 | 76 | 86 | 81 | 76 | 84 | 97 | 87 | 92 | 78 | 93 | 104 | 84 |
| 4 weeks | 85 | 92 | 76 | 78 | 71 | 84 | 94 | 89 | 88 | 88 | 93 | 90 | 95 | 86 |
| 8 weeks | 86 | 94 | 90 | 86 | 73 | 80 | 80 | 91 | 72 | 89 | 72 | 84 | 92 | 87 |
| 16 weeks | 73 | 82 | 94 | 70 | 72 | 89 | 92 | 85 | 69 | 85 | 64 | 69 | 90 | 80 |

Table 15 Adsorption degree (25 °C ) of recombinant 14 valent human papillomavirus vaccine (type 6, 11, 16, 18, 31, 33, 35, 39, 45, 51, 52, 56, 58, 59)

| Lot \ Time \ Type | Adsorption degree(%) | | | | | | | | | | | | | |
|---|---|---|---|---|---|---|---|---|---|---|---|---|---|---|
| | **6** | **11** | **16** | **18** | **31** | **33** | **35** | **39** | **45** | **51** | **52** | **56** | **58** | **59** |
| **Lot1** 0 weeks | 98 | 99 | 100 | >99 | >99 | >99 | >99 | >99 | >99 | >99 | >99 | >99 | >99 | >99 |
| 2 weeks | 99 | 100 | >99 | >99 | >99 | >99 | >99 | >99 | >99 | >99 | >99 | >99 | >99 | >99 |
| 4 weeks | 99 | 99 | >99 | >99 | >99 | 99 | 99 | >99 | >99 | >99 | >99 | 99 | >99 | >99 |
| 8 weeks | >99 | >99 | >99 | >99 | >99 | >99 | >99 | >99 | >99 | >99 | >99 | >99 | >99 | >99 |
| 16 weeks | >99 | >99 | >99 | >99 | >99 | >99 | >99 | >99 | >99 | >99 | >99 | >99 | >99 | >99 |
| **Lot2** 0 weeks | 99 | 100 | 100 | >99 | >99 | >99 | >99 | >99 | 100 | >99 | >99 | >99 | >99 | >99 |
| 2 weeks | >99 | >99 | >99 | >99 | >99 | >99 | >99 | >99 | >99 | >99 | >99 | >99 | >99 | >99 |
| 4 weeks | >99 | >99 | >99 | >99 | >99 | >99 | >99 | >99 | >99 | >99 | >99 | >99 | >99 | >99 |

| | 8 weeks | >99 | >99 | >99 | >99 | >99 | >99 | >99 | >99 | >99 | >99 | >99 | >99 | >99 | >99 |
|---|---|---|---|---|---|---|---|---|---|---|---|---|---|---|---|
| | 16 weeks | >99 | >99 | >99 | >99 | >99 | >99 | >99 | >99 | >99 | >99 | >99 | >99 | >99 | >99 |
| | 0 weeks | 99 | 99 | 99 | >99 | >99 | >99 | >99 | >99 | 99 | >99 | >99 | >99 | >99 | >99 |
| | 2 weeks | 99 | 99 | 99 | 100 | 99 | 99 | >99 | >99 | >99 | >99 | >99 | >99 | >99 | >99 |
| Lot3 | 4 weeks | 100 | 100 | 100 | >99 | >99 | >99 | >99 | >99 | >99 | >99 | >99 | 100 | >99 | >99 |
| | 8 weeks | >99 | >99 | >99 | >99 | >99 | >99 | >99 | >99 | >99 | >99 | >99 | >99 | >99 | >99 |
| | 16 weeks | >99 | >99 | >99 | >99 | >99 | >99 | >99 | >99 | >99 | >99 | >99 | >99 | >99 | >99 |

Table 16 *In vitro* relative potency (4 °C ) of recombinant 14 valent human papillomavirus vaccine (type 6, 11, 16, 18, 31, 33, 35, 39, 45, 51, 52, 56, 58, 59)

| Lot | Time \ Type | *In vitro* relative potency(%) | | | | | | | | | | | | | |
|---|---|---|---|---|---|---|---|---|---|---|---|---|---|---|---|
| | | 6 | 11 | 16 | 18 | 31 | 33 | 35 | 39 | 45 | 51 | 52 | 56 | 58 | 59 |
| Lot1 | 0 months | 95 | 97 | 95 | 107 | 97 | 95 | 107 | 95 | 97 | 103 | 106 | 93 | 97 | 89 |
| | 3 months | 113 | 115 | 100 | 99 | 109 | 103 | 107 | 113 | 112 | 112 | 110 | 132 | 114 | 127 |
| | 6 months | 109 | 102 | 98 | 114 | 114 | 100 | 102 | 105 | 104 | 104 | 100 | 100 | 105 | 102 |
| | 9 months | 125 | 119 | 111 | 108 | 118 | 122 | 115 | 107 | 105 | 98 | 95 | 100 | 92 | 94 |
| | 12 months | 93 | 97 | 92 | 87 | 97 | 94 | 96 | 96 | 102 | 97 | 97 | 100 | 97 | 99 |
| | 18 months | 110 | 102 | 103 | 99 | 100 | 98 | 105 | 100 | 99 | 108 | 99 | 96 | 101 | 104 |
| | 24 months | 113 | 101 | 96 | 95 | 98 | 99 | 95 | 98 | 101 | 95 | 97 | 95 | 96 | 100 |
| Lot2 | 0 months | 103 | 95 | 93 | 114 | 113 | 93 | 118 | 113 | 94 | 88 | 107 | 101 | 113 | 99 |
| | 3 months | 109 | 103 | 94 | 102 | 97 | 86 | 101 | 129 | 106 | 114 | 108 | 116 | 112 | 132 |
| | 6 months | 96 | 88 | 81 | 105 | 109 | 94 | 96 | 111 | 105 | 111 | 95 | 100 | 99 | 108 |
| | 9 months | 123 | 112 | 103 | 106 | 107 | 114 | 110 | 112 | 96 | 101 | 87 | 95 | 95 | 96 |
| | 12 months | 133 | 89 | 92 | 88 | 93 | 92 | 98 | 100 | 89 | 84 | 89 | 86 | 89 | 98 |
| | 18 months | 107 | 94 | 103 | 101 | 98 | 82 | 96 | 107 | 99 | 105 | 99 | 100 | 106 | 117 |
| | 24 months | 110 | 90 | 90 | 92 | 88 | 96 | 95 | 100 | 97 | 99 | 95 | 94 | 100 | 102 |
| Lot3 | 0 months | 90 | 97 | 98 | 105 | 118 | 91 | 115 | 106 | 95 | 93 | 102 | 92 | 118 | 98 |
| | 3 months | 100 | 95 | 95 | 103 | 93 | 76 | 107 | 121 | 104 | 115 | 109 | 92 | 121 | 121 |
| | 6 months | 96 | 96 | 81 | 96 | 124 | 79 | 90 | 98 | 100 | 115 | 98 | 93 | 101 | 104 |
| | 9 months | 113 | 113 | 103 | 99 | 118 | 94 | 103 | 102 | 103 | 97 | 82 | 91 | 93 | 96 |
| | 12 months | 115 | 98 | 100 | 89 | 99 | 78 | 92 | 104 | 109 | 95 | 99 | 88 | 104 | 106 |
| | 18 months | 93 | 96 | 101 | 87 | 96 | 69 | 96 | 102 | 99 | 94 | 97 | 97 | 104 | 110 |
| | 24 months | 103 | 93 | 92 | 89 | 84 | 72 | 91 | 94 | 92 | 80 | 83 | 79 | 97 | 91 |

Table 17 Adsorption degree (4 °C ) of recombinant 14 valent human papillomavirus vaccine (type 6, 11, 16, 18, 31, 33, 35, 39, 45, 51, 52, 56, 58, 59)

| Lot \ Time \ Type | Adsorption degree(%) | | | | | | | | | | | | | |
|---|---|---|---|---|---|---|---|---|---|---|---|---|---|---|
| | 6 | 11 | 16 | 18 | 31 | 33 | 35 | 39 | 45 | 51 | 52 | 56 | 58 | 59 |
| **Lot1** 0 months | 98 | 99 | 100 | >99 | >99 | >99 | >99 | >99 | >99 | >99 | >99 | >99 | >99 | >99 |
| 3 months | 98 | 100 | >99 | >99 | >99 | >99 | >99 | >99 | >99 | >99 | >99 | >99 | >99 | >99 |
| 6 months | 99 | 100 | 100 | >99 | >99 | >99 | >99 | >99 | >99 | >99 | >99 | >99 | >99 | >99 |
| 9 months | 99 | 100 | >99 | >99 | >99 | >99 | >99 | >99 | >99 | >99 | >99 | >99 | >99 | >99 |
| 12 months | 100 | >99 | >99 | >99 | >99 | >99 | >99 | >99 | >99 | >99 | >99 | >99 | >99 | >99 |
| 18 months | >99 | >99 | >99 | >99 | >99 | >99 | >99 | >99 | >99 | >99 | >99 | >99 | >99 | >99 |
| 24 months | >99 | >99 | >99 | >99 | >99 | >99 | >99 | >99 | >99 | >99 | >99 | >99 | >99 | >99 |
| **Lot2** 0months | 99 | 100 | 100 | >99 | >99 | >99 | >99 | >99 | 100 | >99 | >99 | >99 | >99 | >99 |
| 3months | 99 | 100 | >99 | >99 | >99 | >99 | >99 | >99 | >99 | >99 | >99 | >99 | >99 | >99 |
| 6months | 100 | >99 | 100 | >99 | >99 | >99 | >99 | >99 | >99 | >99 | >99 | >99 | >99 | >99 |
| 9months | 100 | 100 | >99 | >99 | >99 | >99 | >99 | >99 | >99 | >99 | >99 | >99 | >99 | >99 |
| 12months | 100 | >99 | 100 | >99 | >99 | >99 | >99 | >99 | >99 | >99 | >99 | >99 | >99 | >99 |
| 18months | >99 | >99 | >99 | >99 | >99 | >99 | >99 | >99 | >99 | >99 | >99 | >99 | >99 | >99 |
| 24months | >99 | >99 | >99 | >99 | >99 | >99 | >99 | >99 | >99 | >99 | >99 | >99 | >99 | >99 |
| **Lot3** 0months | 99 | 99 | 99 | >99 | >99 | >99 | >99 | >99 | 99 | >99 | >99 | >99 | >99 | >99 |
| 3months | 99 | 100 | 100 | >99 | >99 | >99 | >99 | >99 | 100 | >99 | >99 | >99 | >99 | >99 |
| 6months | 100 | 100 | 100 | >99 | >99 | >99 | >99 | >99 | >99 | >99 | >99 | >99 | >99 | >99 |
| 9months | 100 | 100 | 100 | >99 | >99 | >99 | >99 | >99 | >99 | >99 | >99 | >99 | >99 | >99 |
| 12months | 100 | >99 | 100 | >99 | >99 | >99 | >99 | >99 | >99 | >99 | >99 | >99 | >99 | >99 |
| 18months | >99 | >99 | >99 | >99 | >99 | >99 | >99 | >99 | >99 | >99 | >99 | >99 | >99 | >99 |
| 24months | >99 | >99 | >99 | >99 | >99 | >99 | >99 | >99 | >99 | >99 | >99 | >99 | >99 | >99 |

Experimental results show that the 14 valent human papillomavirus vaccine (type 6, 11, 16, 18, 31, 33, 35, 39, 45, 51, 52, 56, 58 and 59) preparation disclosed by the invention has good stability, and can be stably stored for at least 24 months at the temperature of 2 - 8 °C and at least 16 weeks at the temperature of 25 °C.

SEQUENCE LISTING

| SEQ ID NO | Sequence description | Amino acid or nucleic acid sequence |
|---|---|---|
| SEQ ID No: 1 | Amino acid sequence of aa 474-499 of HPV type 33 L1 protein | KAKPKLKRAAPTSTRTSSAKRKKVKK |
| SEQ ID No: 2 | Amino acid sequence of aa 469-499 of HPV type 33 L1 protein | LQAGLKAKPKLKRAAPTSTRTSSAKRKKVKK |
| SEQ ID No: 3 | Amino acid sequence of aa 471-508 of HPV type 59 L1 protein | LQLGARPKPTIGPRKRAAPATSTPSPKRVKRRKSSRK |
| SEQ ID No: 4 | Amino acid sequence of aa 1-469 of HPV type 6 L1 protein | MWRPSDSTVYVPPPNPVSKVVATDAYVTRTNIFYHASSSRLLAVGHPYFSIKRANKTVVPKVS GYQYRVFKVVLPDPNKFALPDSSLFDPTTQRLVWACTGLEVGRGQPLGVGVSGHPFLNKYDD VENSGSGSGNPGQDNRVNVGMDYKQTQLCMVGCAPPLGEHWGKGKQCTNTPVQAGDCPP LELITSVIQDGDMVDTGFGAMNFADLQTNKSDVPIDICGTTCKYPDYLQMAADPYGDRLFFFL RKEQMFARHFFNRAGEVGEPVPDTLIIKGSGNRTSVGSSIYVNTPSGSLVSSEAQLFNKPYWL QKAQGHNNGICWGNQLFVTVVDTTRSTNMTLCASVTSSTYTNSDYKEYMRHVEEYDLQFIF QLCSITLSAEVMAYIHTMNPSVLEDWNFGLSPPPNGTLEDTYRYVQSQAITCQKPTPEKEKPD PYKNLSFWEVNLKEKFSSELDQYPLGRKFLLQSGY |
| SEQ ID No: 5 | Amino acid sequence of aa 1-470 of HPV type 11 L1 protein | MWRPSDSTVYVPPPNPVSKVVATDAYVKRTNIFYHASSSRLLAVGHPYYSIKKVNKTVVPKVS GYQYRVFKVVLPDPNKFALPDSSLFDPTTQRLVWACTGLEVGRGQPLGVGVSGHPLLNKYDD VENSGGYGGNPGQDNRVNVGMDYKQTQLCMVGCAPPLGEHWGKGTQCSNTSVQNGDC PPLELITSVIQDGDMVDTGFGAMNFADLQTNKSDVPLDICGTVCKYPDYLQMAADPYGDRLF FYLRKEQMFARHFFNRAGTVGEPVPDDLLVKGGNNRSSVASSIYVHTPSGSLVSSEAQLFNKP YWLQKAQGHNNGICWGNHLFVTVVDTTRSTNMTLCASVSKSATYTNSDYKEYMRHVEEFDL QFIFQLCSITLSAEVMAYIHTMNPSVLEDWNFGLSPPPNGTLEDTYRYVQSQAITCQKPTPEKE KQDPYKDMSFWEVNLKEKFSSELDQFPLGRKFLLQSGY |
| SEQ ID No: 6 | Amino acid sequence of aa aa 1-474 of HPV type 16 L1 protein | MSLWLPSEATVYLPPVPVSKVVSTDEYVARTNIYYHAGTSRLLAVGHPYFPIKKPNNNKILVPK VSGLQYRVFRIHLPDPNKFGFPDTSFYNPDTQRLVWACVGVEVGRGQPLGVGISGHPLLNKL DDTENASAYAANAGVDNRECISMDYKQTQLCLIGCKPPIGEHWGKGSPCTNVAVNPGDCPP LELINTVIQDGDMVDTGFGAMDFTTLQANKSEVPLDICTSICKYPDYIKMVSEPYGDSLFFYLRR EQMFVRHLFNRAGAVGENVPDDLYIKGSGSTANLASSNYFPTPSGSMVTSDAQIFNKPYWLQ RAQGHNNGICWGNQLFVTVVDTTRSTNMSLCAAISTSETTYKNTNFKEYLRHGEEYDLQFIFQ LCKITLTADVMTYIHSMNSTILEDWNFGLQPPPGGTLEDTYRFVTSQAIACQKHTPPAPKEDPL KKYTFWEVNLKEKFSADLDQFPLGRKFLLQAGL |

| SEQ ID NO | Sequence description | Amino acid or nucleic acid sequence |
|---|---|---|
| SEQ ID No: 7 | Amino acid sequence of aa 1-470 of HPV type 18 L1 protein | MALWRPSDNTVYLPPPSVARVVNTDDYVTRTSIFYHAGSSRLLTVGNPYFRVPAGGGNKQDI PKVSAYQYRVFRVQLPDPNKFGLPDTSIYNPETQRLVWACAGVEIGRGQPLGVGLSGHPFYNK LDDTESSHAATSNVSEDVRDNVSVDYKQTQLCILGCAPAIGEHWAKGTACKSRPLSQGDCPPL ELKNTVLEDGDMVDTGYGAMDFSTLQDTKCEVPLDICQSICKYPDYLQMSADPYGDSMFFCL RREQLFARHFWNRAGTMGDTVPQSLYIKGTGMRASPGSCVYSPSPSGSIVTSDSQLFNKPYW LHKAQGHNNGVCWHNQLFVTVVDTTRSTNLTICASTQSPVPGQYDATKFKQYSRHVEEYDL QFIFQLCTITLTADVMSYIHSMNSSILEDWNFGVPPPPTTSLVDTYRFVQSVAITCQKDAAPAE NKDPYDKLKFWNVDLKEKFSLDLDQYPLGRKFL |
| SEQ ID No: 8 | Amino acid sequence of aa 1-475 of HPV type 31 L1 protein | MSLWRPSEATVYLPPVPVSKVVSTDEYVTRTNIYYHAGSARLLTVGHPYYSIPKSDNPKKIVVPK VSGLQYRVFRVRLPDPNKFGFPDTSFYNPETQRLVWACVGLEVGRGQPLGVGISGHPLLNKF DDTENSNRYAGGPGTDNRECISMDYKQTQLCLLGCKPPIGEHWGKGSPCSNNAITPGDCPPL ELKNSVIQDGDMVDTGFGAMDFTALQDTKSNVPLDICNSICKYPDYLKMVAEPYGDTLFFYLR REQMFVRHFFNRSGTVGESVPTDLYIKGSGSTATLANSTYFPTPSGSMVTSDAQIFNKPYWM QRAQGHNNGICWGNQLFVTVVDTTRSTNMSVCAAIANSDTTFKSSNFKEYLRHGEEFDLQFI FQLCKITLSADIMTYIHSMNPAILEDWNFGLTTPPSGSLEDTYRFVTSQAITCQKSAPQKPKEDP FKDYVFWEVNLKEKFSADLDQFPLGRKFLLQAGY |
| SEQ ID No: 9 | Amino acid sequence of aa 1-472 of HPV type 35 L1 protein | MSLWRSNEATVYLPPVSVSKVVSTDEYVTRTNIYYHAGSSRLLAVGHPYYAIKKQDSNKIAVPK VSGLQYRVFRVKLPDPNKFGFPDTSFYDPASQRLVWACTGVEVGRGQPLGVGISGHPLLNKL DDTENSNKYVGNSGTDNRECISMDYKQTQLCLIGCRPPIGEHWGKGTPCNANQVKAGECPP LELLNTVLQDGDMVDTGFGAMDFTTLQANKSDVPLDICSSICKYPDYLKMVSEPYGDMLFFYL RREQMFVRHLFNRAGTVGETVPADLYIKGTTGTLPSTSYFPTPSGSMVTSDAQIFNKPYWLQR AQGHNNGICWSNQLFVTVVDTTRSTNMSVCSAVSSSDSTYKNDNFKEYLRHGEEYDLQFIFQ LCKITLTADVMTYIHSMNPSILEDWNFGLTPPPSGTLEDTYRVTSQAVTCQKPSAPKPKDDPL KNYTFWEVDLKEKFSADLDQFPLGRKFLLQAGL |

EP 4 292 607 A1

| SEQ ID NO | Sequence description | Amino acid or nucleic acid sequence |
|---|---|---|
| SEQ ID No: 10 | Amino acid sequence of aa 1-469 of HPV type 39 L1 protein | MAMWRSSDSMVYLPPPSVAKVVNTDDYVTRTGIYYYAGSSRLLTVGHPYFKVGMNGGRKQ DIPKVSAYQYRVFRVTLPDPNKFSIPDASLYNPETQRLVWACVGVEVGRGQPLGVGISGHPLY NRQDDTENSPFSSTTNKDSRDNVSVDYKQTQLCIIGCVPAIGEHWGKGKACKPNNVSTGDCP PLELVNTPIEDGDMIDTGYGAMDFGALQETKSEVPLDICQSICKYPDYLQMSADVYGDSMFFC LRREQLFARHFWNRGGMVGDAIPAQLYIKGTDIRANPGSSVYCPSPSGSMVTSDSQLFNKPY WLHKAQGHNNGICWHNQLFLTVVDTTRSTNFTLSTSIESSIPSTYDPSKFKEYTRHVEEYDLQFI FQLCTVTLTTDVMSYIHTMNSSILDNWNFAVAPPPSASLVDTYRYLQSAAITCQKDAPAPEKK DPYDGLKFWNVDLREKFSLELDQFPLGRKFL |
| SEQ ID No: 11 | Amino acid sequence of aa 1-478 of HPV type 45 L1 protein | MALWRPSDSTVYLPPPSVARVVNTDDYVSRTSIFYHAGSSRLLTVGNPYFRVVPSGAGNKQA VPKVSAYQYRVFRVALPDPNKFGLPDSTIYNPETQRLVWACVGMEIGRGQPLGIGLSGHPFYN KLDDTESAHAATAVITQDVRDNVSVDYKQTQLCILGCVPAIGEHWAKGTLCKPAQLQPGDCP PLELKNTIIEDGDMVDTGYGAMDFSTLQDTKCEVPLDICQSICKYPDYLQMSADPYGDSMFFC LRREQLFARHFWNRAGVMGDTVPTDLYIKGTSANMRETPGSCVYSPSPSGSITTSDSQLFNKP YWLHKAQGHNNGICWHNQLFVTVVDTTRSTNLTLCASTQNPVPNTYDPTKFKHYSRHVEEY DLQFIFQLCTITLTAEVMSYIHSMNSSILENWNFGVPPPPTTSLVDTYRFVQSVAVTCQKDTTP PEKQDPYDKLKFWTVDLKEKFSSDLDQYPLGRKFLVQAGL |
| SEQ ID No: 12 | Amino acid sequence of aa 1-474 of HPV type 51 L1 protein | MALWRTNDSKVYLPPAPVSRIVNTEEYITRTGIYYYAGSSRLITLGHPYFPIPKTSTRAAIPKVSAF QYRVFRVQLPDPNKFGLPDPNLYNPDTDRLVWGCVGVEVGRGQPLGVGLSGHPLFNKYDDT ENSRIANGNAQQDVRDNTSVDNKQTQLCIIGCAPPIGEHWGIGTTCKNTPVPPGDCPPLELVS SVIQDGDMIDTGFGAMDFAALQATKSDVPLDISQSVCKYPDYLKMSADTYGNSMFFHLRRE QIFARHYYNKLVGVGEDIPNDYYIKGSGNGRDPIESYIYSATPSGSMITSDSQIFNKPYWLHRAQ GHNNGICWNNQLFITCVDTTRSTNLTISTATAAVSPTFTPSNFKQYIRHGEEYELQFIFQLCKITL TTEVMAYLHTMDPTILEQWNFGLTLPPSASLEDAYRFVRNAATSCQKDTPPQAKPDPLAKYKF WDVDLKERFSLDLDQFALGRKFLLQVGV |
| SEQ ID No: 13 | Amino acid sequence of aa 1-478 of HPV type 52 | MSVWRPSEATVYLPPVPVSKVVSTDEYVSRTSIYYYAGSSRLLTVGHPYFSIKNTSSGNGKKVLV PKVSGLQYRVFRIKLPDPNKFGFPDTSFYNPETQRLVWACTGLEIGRGQPLGVGISGHPLLNKF DDTETSNKYAGKPGIDNRECLSMDYKQTQLCILGCKPPIGEHWGKGTPCNNNSGNPGDCPPL QLINSVIQDGDMVDTGFGCMDFNTLQASKSDVPIDICSSVCKYPDYLQMASEPYGDSLFFFLR |

EP 4 292 607 A1

| SEQ ID NO | Sequence description | Amino acid or nucleic acid sequence |
|---|---|---|
| | L1 protein | REQMFVRHFFNRAGTLGDPVPGDLYIQGSNSGNTATVQSSAFFPTPSGSMVTSESQLFNKPY WLQRAQGHNNGICWGNQLFVTVVDTTRSTNMTLCAEVKKESTYKNENFKEYLRHGEEFDLQ FIFQLCKITLTADVMTYIHKMDATILEDWQFGLTPPPSASLEDTYRFVTSTAITCQKNTPPKGKE DPLKDYMFWEVDLKEKFSADLDQFPLGRKFLLQAGL |
| SEQ ID No: 14 | Amino acid sequence of aa 1-467 of HPV type 56 L1 protein | MATWRPSENKVYLPPTPVSKVVATDSYVKRTSIFYHAGSSRLLAVGHPYYSVTKDNTKTNIPKV SAYQYRVFRVRLPDPNKFGLPDTNIYNPDQERLVWACVGLEVGRGQPLGAGLSGHPLFNRLD DTESSNLANNNVIEDSRDNISVDGKQTQLCIVGCTPAMGEHWTKGAVCKSTQVTTGDCPPLA LINTPIEDGDMIDTGFGAMDFKVLQESKAEVPLDIVQSTCKYPDYLKMSADAYGDSMWFYLR REQLFARHYFNRAGKVGETIPAELYLKGSNGREPPPSSVYVATPSGSMITSEAQLFNKPYWLQR AQGHNNGICWGNQLFVTVVDTTRSTNMTISTATEQLSKYDARKINQYLRHVEEYELQFVFQL CKITLSAEVMAYLHNMNANLLEDWNIGLSPPVATSLEDKYRYVRSTAITCQREQPPTEKQDPL AKYKFWDVNLQDSFSTDLDQFPLGRKFL |
| SEQ ID No: 15 | Amino acid sequence of aa 1-473 of HPV type 58 L1 protein | MSVWRPSEATVYLPPVPVSKVVSTDEYVSRTSIYYYAGSSRLLAVGNPYFSIKSPNNNKKVLVPK VSGLQYRVFRVRLPDPNKFGFPDTSFYNPDTQRLVWACVGLEIGRGQPLGVGVSGHPYLNKF DDTETSNRYPAQPGSDNRECLSMDYKQTQLCLIGCKPPTGEHWGKGVACNNNAAATDCPPL ELFNSIIEDGDMVDTGFGCMDFGTLQANKSDVPIDICNSTCKYPDYLKMASEPYGDSLFFFLRR EQMFVRHFFNRAGKLGEAVPDDLYIKGSGNTAVIQSSAFFPTPSGSIVTSESQLFNKPYWLQR AQGHNNGICWGNQLFVTVVDTTRSTNMTLCTEVTKEGTYKNDNFKEYVRHVEEYDLQFVFQ LCKITLTAEIMTYIHTMDSNILEDWQFGLTPPPSASLQDTYRFVTSQAITCQKTAPPKEKEDPLN KYTFWEVNLKEKFSADLDQFPLGRKFLLQSGL |
| SEQ ID No: 16 | Amino acid sequence of chimeric HPV type 6 L1 protein | MWRPSDSTVYVPPPNPVSKVVATDAYVTRTNIFYHASSSRLLAVGHPYFSIKRANKTVVPKVS GYQYRVFKVVLPDPNKFALPDSSLFDPTTQRLVWACTGLEVGRGQPLGVGVSGHPFLNKYDD VENSGSGGNPGQDNRVNVGMDYKQTQLCMVGCAPPLGEHWGKGKQCTNTPVQAGDCPP LELITSVIQDGDMVDTGFGAMNFADLQTNKSDVPIDICGTTCKYPDYLQMAADPYGDRLFFFL RKEQMFARHFFNRAGEVGEPVPDTLIIKGSGNRTSVGSSIYVNTPSGSLVSSEAQLFNKPYWL QKAQGHNNGICWGNQLFVTVVDTTRSTNMTLCASVTTSSTYTNSDYKEYMRHVEEYDLQFIF QLCSITLSAEVMAYIHTMNPSVLEDWNFGLSPPPNGTLEDTYRYVQSQAITCQKPTPEKEKPD PYKNLSFWEVNLKEKFSSELDQYPLGRKFLLQSGYKAKPKLKRAAPTSTRTSSAKRKKVKK |

19

| SEQ ID NO | Sequence description | Amino acid or nucleic acid sequence |
|---|---|---|
| SEQ ID No: 17 | Amino acid sequence of chimeric HPV type 11 L1 protein | MWRPSDSTVYVPPPNPVSKVVATDAYVKRTNIFYHASSSRLLAVGHPYYSIKKVNKTVVPKVS GYQYRVFKVVLPDPNKFALPDSSLFDPTTQRLVWACTGLEVGRGQPLGVGVSGHPLLNKYDD VENSGGYGGNPGQDNRVNVGMDYKQTQLCMVGCAPPLGEHWGKGTQCSNTSVQNGDC PPLELITSVIQDGDMVDTGFGAMNFADLQTNKSDVPLDICGTVCKYPDYLQMAADPYGDRLF FYLRKEQMFARHFFNRAGTVGEPVPDDLLVKGGNNRSSVASSIYVHTPSGSLVSSEAQLFNKP YWLQKAQGHNNGICWGNHLFVTVVDTTRSTNMTLCASVSKSATYTNSDYKEYMRHVEEFDL QFIFQLCSITLSAEVMAYIHTMNPSVLEDWNFGLSPPPNGTLEDTYRYVQSQAITCQKPTPEKE KQDPYKDMSFWEVNLKEKFSSELDQFPLGRKFLLQSGYKAKPKLKRAAPTSTRTSSAKRKKVKK |
| SEQ ID No: 18 | Amino acid sequence of chimeric HPV type 16 L1 protein | MSLWLPSEATVYLPPVPVSKVVSTDEYVARTNIYYHAGTSRLLAVGHPYFPIKKPNNNKILVPK VSGLQYRVFRIHLPDPNKFGFPDTSFYNPDTQRLVWACVGVEVGRGQPLGVGISGHPLLNKL DDTENASAYAANAGVDNRECISMDYKQTQLCLIGCKPPIGEHWGKGSPCTNVAVNPGDCPP LELINTVIQDGDMVDTGFGAMDFTTLQANKSEVPLDICTSICKYPDYIKMVSEPYGDSLFFYLRR EQMFVRHLFNRAGAVGENVPDDLYIKGSGSTANLASSNYFPTPSGSMVTSDAQIFNKPYWLQ RAQGHNNGICWGNQLFVTVVDTTRSTNMSLCAAISTSETTYKNTNFKEYLRHGEEYDLQFIFQ LCKITLTADVMTYIHSMNSTILEDWNFGLQPPPGGTLEDTYRFVTSQAIACQKHTPPAPKEDPL KKYTFWEVNLKEKFSADLDQFPLGRKFLLQAGLKAKPKLKRAAPTSTRTSSAKRKKVKK |
| SEQ ID No: 19 | Amino acid sequence of chimeric HPV type 18 L1 protein | MALWRPSDNTVYLPPPSVARVVNTDDYVTRTSIFYHAGSSRLLTVGNPYFRVPAGGGNKQDI PKVSAYQYRVFRVQLPDPNKFGLPDTSIYNPETQRLVWACAGVEIGRGQPLGVGLSGHPFYNK LDDTESSHAATSNVSEDVRDNVSVDYKQTQLCILGCAPAIGEHWAKGTACKSRPLSQGDCPPL ELKNTVLEDGDMVDTGYGAMDFSTLQDTKCEVPLDICQSICKYPDYLQMSADPYGDSMFFCL RREQLFARHFWNRAGTMGDTVPQSLYIKGTGMRASPGSCVYSPSPSGSIVTSDSQLFNKPYW LHKAQGHNNGVCWHNQLFVTVVDTTRSTNLTICASTQSPVPGQYDATKFKQYSRHVEEYDL QFIFQLCTITLTADVMSYIHSMNSSILEDWNFGVPPPPTTSLVDTYRFVQSVAITCQKDAAPAE NKDPYDKLKFWNVDLKEKFSLDLDQYPLGRKFLKAKPKLKRAAPTSTRTSSAKRKKVKK |
| SEQ ID | Amino acid sequence of | MSLWRPSEATVYLPPVPVSKVVSTDEYVTRTNIYYHAGSARLLTVGHPYYSIPKSDNPKKIVVPK VSGLQYRVFRVRLPDPNKFGFPDTSFYNPETQRLVWACVGLEVGRGQPLGVGISGHPLLNKF |

EP 4 292 607 A1

(continued)

| SEQ ID NO | Sequence description | Amino acid or nucleic acid sequence |
|---|---|---|
| No: 20 | chimeric HPV type 31 L1 protein | DDTENSNRYAGGPGTDNRECISMDYKQTQLCLLGCKPPIGEHWGKGSPCSNNAITPGDCPPL ELKNSVIQDGDMVDTGFGAMDFTALQDTKSNVPLDICNSICKYPDYLKMVAEPYGDTLFFYLR REQMFVRHFFNRSGTVGESVPTDLYIKGSGSTATLANSTYFPTPSGSMVTSDAQIFNKPYWM QRAQGHNNGICWGNQLFVTVVDTTRSTNMSVCAAIANSDTTFKSSNFKEYLRHGEEFDLQFI FQLCKITLSADIMTYIHSMNPAILEDWNFGLTTPPSGSLEDTYRFVTSQAITCQKSAPQKPKEDP FKDYVFWEVNLKEKFSADLDQFPLGRKFLLQAGYKAKPKLKRAAPTSTRTSSAKRKKVKK |
| SEQ ID No: 21 | Amino acid sequence of chimeric HPV type 35 L1 protein | MSLWRSNEATVYLPPVSVSKVVSTDEYVTRTNIYYHAGSSRLLAVGHPYYAIKKQDSNKIAVPK VSGLQYRVFRVKLPDPNKFGFPDTSFYDPASQRLVWACTGVEVGRGQPLGVGISGHPLLNKL DDTENSNKYVGNSGTDNRECISMDYKQTQLCLIGCRPPIGEHWGKGTPCNANQVKAGECPP LELLNTVLQDGDMVDTGFGAMDFTTLQANKSDVPLDICSSICKYPDYLKMVSEPYGDMLFFYL RREQMFVRHLFNRAGTVGETVPADLYIKGTTGTLPSTSYFPTPSGSMVTSDAQIFNKPYWLQR AQGHNNGICWSNQLFVTVVDTTRSTNMSVCSAVSSSDSTYKNDNFKEYLRHGEEYDLQFIFQ LCKITLTADVMTYIHSMNPSILEDWNFGLTPPPSGTLEDTYRYVTSQAVTCQKPSAPKPKDDPL KNYTFWEVDLKEKFSADLDQFPLGRKFLLQAGLKAKPKLKRAAPTSTRTSSAKRKKVKK |
| SEQ ID No: 22 | Amino acid sequence of chimeric HPV type 39 L1 protein | MAMWRSSDSMVYLPPPSVAKVVNTDDYVTRTGIYYAGSSRLLTVGHPYFKVGMNGGRKQ DIPKVSAYQYRVFRVTLPDPNKFSIPDASLYNPETQRLVWACVGVEVGRGQPLGVGISGHPLY NRQDDTENSPFSSTTNKDSRDNVSVDYKQTQLCIIGCVPAIGEHWGKGACKPNNVSTGDCP PLELVNTPIEDGDMIDTGYGAMDFGALQETKSEVPLDICQSICKYPDYLQMSADVVGDSMFFC LRREQLFARHFWNRGGMVGDAIPAQLYIKGTDIRANPGSSVYCPSPSGSMVTSDSQLFNKPY WLHKAQGHNNGICWHNQLFLTVVDTTRSTNFTLSTSIESSIPSTYDPSKFKEYTRHVEEYDLQFI FQLCTVTLTDVMSYIHTMNSSILDNWNFAVAPPPSASLVDTYRYLQSAAITCQKDAPAPEKK DPYDGLKFWNVDLREKFSLELDQFPLGRKFLLQLGARPKPTIGPRKRAAPAPTSTPSPKRVKRR KSSRK |
| SEQ ID No: 23 | Amino acid sequence of chimeric HPV type 45 L1 protein | MALWRPSDSTVYLPPPSVARVVNTDDYVSRTSIFYHAGSSRLLTVGNPYFRVVPSGAGNKQA VPKVSAYQYRVFRVALPDPNKFGLPDSTIYNPETQRLVWACVGMEIGRGQPLGIGLSGHPFYN KLDDTESAHAATAVITQDVRDNVSVDYKQTQLCILGCVPAIGEHWAKGTLCKPAQLQPGDCP PLELKNTIIEDGDMVDTGYGAMDFSTLQDTKCEVPLDICQSICKYPDYLQMSADPYGDSMFFC LRREQLFARHFWNRAGVMGDTVPTDLYIKGTSANMRETPGSCVYSPSPSGSIITTSDSQLFNKP YWLHKAQGHNNGICWHNQLFVTVVDTTRSTNLTLCASTQNPVPNTYDPTKFKHYSRHVEEY DLQFIFQLCTITLTAEVMSYIHSMNSSILENWNFGVPPPPTSLVDTYRFVQSVAVTCQKDTTP PEKQDPYDKLKFWTVDLKEKFSSDLDQYPLGRKFLVQAGLKAKPKLKRAAPTSTRTSSAKRKKV KK |

(continued)

| SEQ ID NO | Sequence description | Amino acid or nucleic acid sequence |
|---|---|---|
| SEQ ID No: 24 | Amino acid sequence of chimeric HPV type 51 L1 protein | MALWRTNDSKVYLPPAPVSRIVNTEEYITRTGIYYYAGSSRLITLGHPYFPIPKTSTRAAIPKVSAF QYRVFRVQLPDPNKFGLPDPNLYNPDTDRLVWGCVGVEVGRGQPLGVGLSGHPLFNKYDDT ENSRIANGNAQQDVRDNTSVDNKQTQLCIIGCAPPIGEHWGIGTTCKNTPVPPGDCPPLELVS SVIQDGDMIDTGFGAMDFAALQATKSDVPLDISQSVCKYPDYLKMSADTYGNSMFFHLRRE QIFARHYYNKLVGVGEDIPNDYYIKGSGNGRDPIESYIYSATPSGSMITSDSQIFNKPYWLHRAQ GHNNGICWNNQLFITCVDTTRSTNLTISTATAAVSPTFTPSNFKQYIRHGEEYELQFIFQLCKITL TTEVMAYLHTMDPTILEQWNFGLTLPPSASLEDAYRFVRNAATSCQKDTPPQAKPDPLAKYKF WDVDLKERFSLDLDQFALGRKFLLQVGVKAKPKLKRAAPTSTRTSSAKRKKVKK |
| SEQ ID No:2 5 | Amino acid sequence of chimeric HPV type 52 L1 protein | MSVWRPSEATVYLPPVPVSKVVSTDEYVSRTSIYYYAGSSRLLTVGHPYFSIKNTSSGNGKKVLV PKVSGLQYRVFRIKLPDPNKFGFPDTSFYNPETQRLVWACTGLEIGRGQPLGVGISGHPLLNKF DDTETSNKYAGKPGIDNRECLSMDYKQTQLCILGCKPPIGEHWGKGTPCNNNSGNPGDCPPL QLINSVIQDGDMVDTGFGCMDFNTLQASKSDVPIDICSSVCKYPDYLQMASEPYGDSLFFFLR REQMFVRHFFNRAGTLGDPVPGDLYIQGSNSGNTATVQSSAFFPTPSGSMVTSESQLFNKPY WLQRAQGHNNGICWGNQLFVTVVDTTRSTNMTLCAEVKKESTYKNENFKEYLRHGEEFDLQ FIFQLCKITLTADVMTYIHKMDATILEDWQFGLTPPPSASLEDTYRFVTSTAITCQKNTPPKGKE DPLKDYMFWEVDLKEKFSADLDQFPLGRKFLLQAGLKAKPKLKRAAPTSTRTSSAKRKKVKK |
| SEQ ID No: 26 | Amino acid sequence of chimeric HPV type 56 L1 protein | MATWRPSENKVYLPPTPVSKVVATDSYVKRTSIFYHAGSSRLLAVGHPYYSVTKDNTKTNIPKV SAYQYRVFRVRLPDPNKFGLPDTNIYNPDQERLVWACVGLEVGRGQPLGAGLSGHPLFNRLD DTESSNLANNNVIEDSRDNISVDGKQTQLCIVGCTPAMGEHWTKGAVCKSTQVTTGDCPPLA LINTPIEDGDMIDTGFGAMDFKVLQESKAEVPLDIVQSTCKYPDYLKMSADAYGDSMWFYLR REQLFARHYFNRAGKVGETIPAELYLKGSNGREPPPSSVYVATPSGSMITSEAQLFNKPYWLQR AQGHNNGICWGNQLFVTVVDTTRSTNMTISTATEQLSKYDARKINQYLRHVEEYELQFVFQL |
| | | CKITLSAEVMAYLHNMNANLLEDWNIGLSPPVATSLEDKYRYVRSTAITCQREQPPTEKQDPL AKYKFWDVNLQDSFSTDLDQFPLGRKFLLQAGLKAKPKLKRAAPTSTRTSSAKRKKVKK |

EP 4 292 607 A1

22

(continued)

| SEQ ID NO | Sequence description | Amino acid or nucleic acid sequence |
|---|---|---|
| SEQ ID No: 27 | Amino acid sequence of chimeric HPV type 58 L1 protein | MSVWRPSEATVYLPPVPVSKVVSTDEYVSRTSIYYAGSSRLLAVGNPYFSIKSPNNNKKVLVPK VSGLQYRVFRVRLPDPNKFGFPDTSFYNPDTQRLVWACVGLEIGRGQPLGVGVSGHPYLNKF DDTETSNRYPAQPGSDNRECLSMDYKQTQLCLIGCKPPTGEHWGKGVACNNNAAATDCPPL ELFNSIIEDGDMVDTGFGCMDFGTLQANKSDVPIDICNSTCKYPDYLKMASEPYGDSLFFFLRR EQMFVRHFFNRAGKLGEAVPDDLYIKGSGNTAVIQSSAFFPTPSGSIVTSESQLFNKPYWLQR AQGHNNGICWGNQLFVTVVDTTRSTNMTLCTEVTKEGTYKNDNFKEYVRHVEEYDLQFVFQ LCKITLTAEIMTYIHTMDSNILEDWQFGLTPPPSASLQDTYRFVTSQAITCQKTAPPKEKEDPLN KYTFWEVNLKEKFSADLDQFPLGRKFLLQSGLKAKPKLKRAAPTSTRTSSAKRKKVKK |
| SEQ ID No: 28 | Amino acid sequence of HPV type 33 L1 protein | MSVWRPSEATVYLPPVPVSKVVSTDEYVSRTSIYYAGSSRLLAVGHPYFSIKNPTNAKKLLVPK VSGLQYRVFRVRLPDPNKFGFPDTSFYNPDTQRLVWACVGLEIGRGQPLGVGISGHPLLNKFD DTETGNKYPGQPGADNRECLSMDYKQTQLCLLGCKPPTGEHWGKGVACTNAAPANDCPPL ELINTIIEDGDMVDTGFGCMDFKTLQANKSDVPIDICGSTCKYPDYLKMTSEPYGDSLFFFLRRE QMFVRHFFNRAGTLGEAVPDDLYIKGSGTTASIQSSAFFPTPSGSMVTSESQLFNKPYWLQRA QGHNNGICWGNQVFVTVVDTTRSTNMTLCTQVTSDSTYKNENFKEYIRHVEEYDLQFVFQLC KVTLTAEVMTYIHAMNPDILEDWQFGLTPPPSASLQDTYRFVTSQAITCQKTVPPKEKEDPLG KYTFWEVDLKEKFSADLDQFPLGRKFLLQAGLKAKPKLKRAAPTSTRTSSAKRKKVKK |
| SEQ ID No: 29 | Amino acid sequence of HPV type 59 L1 protein | MALWRSSDNKVYLPPPSVAKVVSTDEYVTRTSIFYHAGSSRLLTVGHPYFKVPKGGNGRQDVP KVSAYQYRVFRVKLPDPNKFGLPDNTVVDPNSQRLVWACVGVEIGRGQPLGVGLSGHPLYNK LDDTENSHVASAVDTKDTRDNVSVDYKQTQLCIIGCVPAIGEHWTKGTACKPTTVVQGDCPP LELINTPIEDGDMVDTGYGAMDFKLLQDNKSEVPLDICQSICKYPDYLQMSADAYGDSMFFCL RREQVFARHFWNRSGTMGDQLPESLYIKGTDIRANPGSYLYSPSPSGSVVTSDSQLFNKPYWL HKAQGLNNGICWHNQLFLTVVDTTRSTNLSVCASTTSSIPNVYTPTSFKEYARHVEEFDLQFIF QLCKITLTTEVMSYIHNMNTTILEDWNFGVTPPTASLVDTYRFVQSAAVTCQKDTAPPVKQD PYDKLKFWPVDLKERFSADLDQFPLGRKFLLQLGARPKPTIGPRKRAAPAPTSTPSPKRVKRRK SSRK |

23

(continued)

| SEQ ID NO | Sequence description | Amino acid or nucleic acid sequence |
|---|---|---|
| SEQ ID No: 30 | Synthetic HPV6L1 gene | ctgggtaccATGTGGAGACCATCTGACAGCACAGTCTATGTGCCTCCTCCAAACCCTGTGAGCAAGGTGGTGGCTACAGATGCCTATGTGACCAGGACCAACATCTTCTACCATGCCTCCTCCAGCAGACTGCTGGCTGTGGGACACCCATACTTCAGCATCAAGAGGGCTAACAAGACAGTGGTGCCAAAGGTGTCTGGCTACCAATACAGGGTGTTCAAGGTGGTGCTGCCTGACCCAAACAAGTTTGCCCTGCCTGACTCCTCCCTGTTTGACCCAACCACCCAGAGACTGGTGTGGGCTTGTACTGGATTGGAGGTGGGCAGGGGACAACCACTGGGAGTGGGAGTGTCTGGACACCCATTCCTGAACAAATATGATGATGTGGAGAACTCTGGCTCTGGAGGCAACCCTGGACAAGACAACAGGGTGAATGTGGGGGATGGACTACAAGCAGACCCAACTTTGTATGGTGGGCTGTGCCCCTCCACTGGGAGAACACTGGGGCAAGGGCAAGCAGTGTACCAACACACCTGTCCAGGCTGGAGACTGTCCTCCATTGGAACTGATTACCTCTGTGATTCAGGATGGAGATATGGTGGACACAGGCTTTGGAGCTATGAACTTTGCTGACCTCCAAACCAACAAGTCTGATGTGCCAATTGACATCTGTGGCACCACTTGTAAATACCCTGACTACCTCCAAATGGCTGCTGACCCATATGGAGACAGACTGTTCTTCTTCCTGAGGAAGGAACAGATGTTTGCCAGACACTTCTTCAACAGGGCTGGAGAGGTGGGAGAACCTGTGCCTGACACCCTGATTATCAAGGGCTCTGGCAACAGGACCTCTGTGGGCTCCAGCATCTATGTGAACACACCATCTGGCTCCCTGGTGTCCTCTGAGGCTCAACTTTTCAACAAGCCATACTGGCTCCAAAAGGCTCAAGGACACAACAATGGCATCTGTTGGGGCAACCAACTTTTTGTGACAGTGGTGGACACCACCAGGAGCACCAATATGACCCTGTGTGCCTCTGTGACCACCTCCAGCACCTACACCAACTCTGACTACAAGGAATATATGAGGCATGTGGAGGAATATGACCTCCAATTCATCTTCCAACTTTGTAGCATCACCCTGTCTGCTGAGGTGATGGCTTACATCCACACAATGAACCCATCTGTGTTGGAGGACTGGAACTTTGGACTGAGCCCTCCTCCAAATGGCACCTTGGAGGACACCTACAGATATGTCCAGAGCCAGGCTATCACTTGTCAGAAGCCAACACCTGAGAAGGAGAAGCCTGACCCATACAAGAACCTGTCCTTCTGGGAGGTGAACCTGAAAGAGAAGTTCTCCTCTGAACTGGACCAATACCCACTGGGCAGGAAGTTCCTGCTCCAATCTGGCTACAGGGGCAGGTCCAGCATCAGGACAGGAGTGAAGAGACCTGCTGTGAGCAAGGCATCTGCTGCCCCAAAGAGGAAGAGGGCTAAGACCAAGAGGTAAActcgagctc |
| SEQ | Synthetic | ctgggtaccATGTGGAGACCATCTGACAGCACAGTCTATGTGCCTCCTCCAAACCCTGTGAGC |

| SEQ ID NO | Sequence description | Amino acid or nucleic acid sequence |
|---|---|---|
| ID No: 31 | HPV11L1 gene Synthetic HPV 11 L1 gene | AAGGTGGTGGCTACAGATGCCTATGTGAAGAGGACCAACATCTTCTACCATGCCTCCTCCA GCAGACTGCTGGCTGTGGGACACCCATACTACAGCATCAAGAAGGTGAACAAGACAGTG GTGCCAAAGGTGTCTGGCTACCAATACAGGGTGTTCAAGGTGGTGCTGCCTGACCCAAAC AAGTTTGCCCTGCCTGACTCCTCCCTGTTTGACCCAACCACCCAGAGACTGGTGTGGGCTT GTACTGGATTGGAGGTGGGCAGGGGACAACCACTGGGAGTGGGAGTGTCTGGACACCCA CTGCTGAACAAATATGATGATGTGGAGAACTCTGGAGGCTATGGAGGCAACCCTGGACAA GACAACAGGGTGAATGTGGGGGATGGACTACAAGCAGACCCAACTTTGTATGGTGGGCTG TGCCCCTCCACTGGGAGAACACTGGGGCAAGGGCACCCAGTGTAGCAACACCTCTGTCCA GAATGGAGACTGTCCTCCATTGGAACTGATTACCTCTGTGATTCAGGATGGAGATATGGT GGACACAGGCTTTGGAGCTATGAACTTTGCTGACCTCCAAACCAACAAGTCTGATGTGCCA CTGGACATCTGTGGCACAGTGTGTAAATACCCTGACTACCTCCAAATGGCTGCTGACCCAT ATGGAGACAGACTGTTCTTCTACCTGAGGAAGGAACAGATGTTTGCCAGACACTTCTTCAA CAGGGCTGGCACAGTGGGAGAACCTGTGCCTGATGACCTGCTGGTGAAGGGAGGCAACA ACAGGTCCTCTGTGGCATCCAGCATCTATGTGCATACACCATCGGCTCCCTGGTGTCCTCT GAGGCTCAACTTTTCAACAAGCCATACTGGCTCCAAAAGGCTCAAGGACACAACAATGGC ATCTGTTGGGGCAACCACCTGTTTGTGACAGTGGTGGACACCACCAGGAGCACCAATATG ACCCTGTGTGCCTCTGTGAGCAAGTCTGCCACCTACACCAACTCTGACTACAAGGAATATA TGAGGCATGTGGAGGAGTTTGACCTCCAATTCATCTTCCAACTTTGTAGCATCACCCTGTCT GCTGAGGTGATGGCTTACATCCACACAATGAACCCATCTGTGTTGGAGGACTGGAACTTT GGACTGAGCCCTCCTCCAAATGGCACCTTGGAGGACACCTACAGATATGTCCAGAGCCAG GCTATCACTTGTCAGAAGCCAACACCTGAGAAGGAGAAGCAGGACCCATACAAGGATATG AGTTTCTGGGAGGTGAACCTGAAAGAGAAGTTCTCCTCTGAACTGGACCAGTTTCCACTG GGCAGGAAGTTCCTGCTCCAATCTGGCTACAGGGGCAGGACCTCTGCCAGGACAGGCATC AAGAGACCTGCTGTGAGCAAGCCAAGCACAGCCCCAAAGAGGAAGAGGACCAAGACCAA GAAGTAAActcgagctc |

| SEQ ID NO | Sequence description | Amino acid or nucleic acid sequence |
|---|---|---|
| SEQ ID No: 32 | Synthetic HPV16L1 gene | ctgggtaccATGAGTCTGTGGCTGCCATCTGAGGCTACAGTCTACCTGCCTCCTGTGCCTGTG AGCAAGGTGGTGAGCACAGATGAATATGTGGCAAGGACCAACATCTACTACCATGCTGGC ACCAGCAGACTGCTGGCTGTGGGACACCCATACTTTCCAATCAAGAAGCCAAACAACAAC AAGATTCTGGTGCCAAAGGTGTCTGGACTCCAATACAGGGTGTTCAGGATTCACCTGCCT GACCCAAACAAGTTTGGCTTTCCTGACACCTCCTTCTACAACCCTGACACCCAGAGACTGG TGTGGGCTTGTGTGGGAGTGGAGGTGGGCAGGGGACAACCACTGGGAGTGGGCATCTCT GGACACCCACTGCTGAACAAACTGGATGACACAGAGAATGCCTCTGCCTATGCTGCCAAT GCTGGAGTGGACAACAGGGAGTGTATCAGTATGGACTACAAGCAGACCCAACTTTGTCTG ATTGGCTGTAAGCCTCCAATTGGAGAACACTGGGGCAAGGGCAGCCCATGTACCAATGTG GCTGTGAACCCTGGAGACTGTCCTCCATTGGAACTGATAAACACAGTGATTCAGGATGGA GATATGGTGGACACAGGCTTTGGAGCTATGGACTTCACCACCCTCCAAGCCAACAAGTCT GAGGTGCCACTGGACATCTGTACCAGCATCTGTAAATACCCTGACTACATCAAGATGGTGT CTGAACCATATGGAGACTCCCTGTTCTTCTACCTGAGGAGGGAACAGATGTTTGTGAGAC ACCTGTTCAACAGGGCTGGAGCAGTGGGAGAGAATGTGCCTGATGACCTCTACATCAAGG GCTCTGGCAGCACAGCCAACCTGGCATCCAGCAACTACTTTCCAACACCATCTGGCAGTAT GGTGACCTCTGATGCCCAGATTTTCAACAAGCCATACTGGCTCCAAAGGGCTCAAGGACA CAACAATGGCATCTGTTGGGGCAACCAACTTTTTGTGACAGTGGTGGACACCACCAGGAG CACCAATATGAGTCTGTGTGCTGCCATCAGCACCTCTGAGACCACCTACAAGAACACCAAC TTCAAGGAATACCTGAGACATGGAGAGGAATATGACCTCCAATTCATCTTCCAACTTTGTA AGATTACCCTGACAGCAGATGTGATGACCTACATCCACAGTATGAACAGCACCATCTTGGA GGACTGGAACTTTGGACTCCAACCTCCTCCTGGAGGCACCTTGGAGGACACCTACAGGTTT GTGACCAGCCAGGCTATTGCCTGTCAGAAACACACACCTCCTGCCCCAAAGGAGGACCCA CTGAAAAAATACACCTTCTGGGAGGTGAACCTGAAAGAGAAGTTCTCTGCTGACCTGGAC CAGTTTCCACTGGGCAGGAAGTTCCTGCTCCAAGCAGGACTGAAAGCCAAGCCAAAGTTC ACCCTGGGCAAGAGGAAGGCTACACCAACCACCTCCAGCACCAGCACCACAGCCAAGAG GAAGAAGAGGAAACTGTAAActcgagctc |
| SEQ ID No: | Synthetic HPV18L1 gene | ctgggtaccATGGCCCTCTGGAGACCATCCGATAACACAGTGTACTTGCCCCCCACCCAGCGTC GCCCGGGTGGTGAACACAGACGACTACGTCACCAGAACCTCAATCTTCTACCACGCCGGG TCCAGCCGGCTGCTGACCGTGGGCAACCCCTACTTCCGCGTGCCCGCCGGCGGCGGAAAC |

| SEQ ID NO | Sequence description | Amino acid or nucleic acid sequence |
|---|---|---|
| 33 | | AAACAAGACATCCCCAAAGTCAGCGCCTATCAGTACCGGGTGTTCCGCGTCCAACTGCCCG ATCCCAACAAGTTCGGCCTGCCCGACACCTCCATCTACAACCCCGAGACCCAGAGGCTGGT CTGGGCTTGCGCCGGCGTCGAGATCGGGAGGGGCCAACCCCTGGGCGTGGGGTTGTCCG GCCACCCCTTCTACAACAAGCTGGACGATACCGAGTCCAGCCACGCAGCAACCAGCAACG TCTCCGAAGATGTGCGCGATAACGTCAGCGTGGACTACAAACAAACCCAACTGTGCATCCT GGGATGCGCACCCGCCATCGGCGAGCATTGGGCCAAGGGGACCGCCTGCAAGAGCAGGC CCCTGAGCCAAGGGGACTGTCCACCCCTGGAGTTGAAGAATACCGTGCTCGAGGACGGC GACATGGTGGACACCGGCTACGGCGCTATGGATTTCTCCACCCTCCAGGACACCAAGTGC GAAGTGCCCCTCGACATCTGCCAAAGCATCTGCAAGTACCCCGACTACCTCCAGATGAGCG CCGACCCCTACGGCGACAGCATGTTCTTCTGTCTCAGAAGGGAACAATTGTTCGCCCGCCA CTTCTGGAACCGGGCCGGCACAATGGGAGATACAGTCCCCCAGAGCCTGTACATCAAGGG GACCGGAATGAGGGCCAGCCCCGGGTCCTGCGTCTACAGCCCAAGCCCCTCCGGGAGCAT CGTCACAAGCGATAGCCAACTCTTCAACAAGCCCTACTGGCTCCACAAAGCCCAAGGCCAC AATAACGGGGTGTGTTGGCACAACCAGCTGTTCGTGACCGTCGTGGACACAACCAGGTCC ACAAACCTGACCATCTGCGCCAGCACCCAAAGCCCCGTGCCCGGCCAGTACGACGCCACA AAGTTCAAACAATACTCTCGGCACGTGGAAGAGTACGACCTCCAATTCATCTTCCAACTCT GCACCATCACCCTCACCGCCGACGTGATGAGCTACATCCACTCCATGAACTCCTCCATCCTG GAAGACTGGAATTTCGGCGTGCCACCACCCCCTACCACCTCCCTCGTCGACACCTACAGAT TCGTGCAGAGCGTGGCCATCACATGCCAGAAAGACGCCGCCCCCGCCGAGAACAAAGAC CCATACGACAAACTGAAATTCTGGAACGTCGACCTGAAAGAGAAATTCAGCCTGGATCTG GACCAGTACCCATTGGGCAGGAAGTTCCTCGTGCAAGCCGGCCTCAGGAGAAAACCAACA ATCGGGCCCAGGAAGAGGAGCGCCCCCAGCGCAACCACCAGCAGCAAGCCCGCAAAAAG GGTCAGAGTGAGGGCACGCAAATAAActcgagctc |

EP 4 292 607 A1

| SEQ ID NO | Sequence description | Amino acid or nucleic acid sequence |
|---|---|---|
| SEQ ID No: 34 | Synthetic HPV31L1 gene | ctgggtaccATGAGCCTGTGGAGGCCCAGCGAGGCCACCGTGTACCTGCCCCCCGTGCCCGTGAGCAAGGTGGTGAGCACCGACGAGTACGTGACCAGGACCAACATCTACTACCACGCCGGCAGCGCCAGGCTGCTGACCGTGGGCCACCCCTACTACAGCATCCCCAAGAGCGACAACCCCAAGAAGATCGTGGTGCCCAAGGTGAGCGGCCTGCAGTACAGGGTGTTCAGGGTGAGGCTGCCCGACCCCAACAAGTTCGGCTTCCCCGACACCAGCTTCTACAACCCCGAGACCCAGAGGCTGGTGTGGGCCTGCGTGGGCCTGGAGGTGGGCAGGGGCCAGCCCCTGGGCGTGGGCATCAGCGGCCACCCCCTGCTGAACAAGTTCGACGACACCGAGAACAGCAACAGGTACGCCGGCGGCCCCGGCACCGACAACAGGGAGTGCATCAGCATGGACTACAAGCAGACCCAGCTGTGCCTGCTGGGCTGCAAGCCCCCCATCGGCGAGCACTGGGGCAAGGGCAGCCCCTGCAGCAACAACGCCATCACCCCCGGCGACTGCCCCCCCCTGGAGCTGAAGAACAGCGTGATCCAGGACGGCGACATGGTGGACACCGGCTTCGGCGCCATGGACTTCACCGCCCTGCAGGACACCAAGAGCAACGTGCCCCTGGACATCTGCAACAGCATCTGCAAGTACCCCGACTACCTGAAGATGGTGGCCGAGCCCTACGGCGACACCCTGTTCTTCTACCTGAGGAGGGAGCAGATGTTCGTGAGGCACTTCTTCAACAGGAGCGGCACCGTGGGCGAGAGCGTGCCCACCGACCTGTACATCAAGGGCAGCGGCAGCACCGCCACCCTGGCCAACAGCACCTACTTCCCCACCCCCAGCGGCAGCATGGTGACCAGCGACGCCCAGATCTTCAACAAGCCCTACTGGATGCAGAGGGCCCAGGGCCACAACAACGGCATCTGCTGGGGCAACCAGCTGTTCGTGACCGTGGTGGACACCACCAGGAGCACCAACATGAGCGTGTGCGCCGCCATCGCCAACAGCGACACCACCTTCAAGAGCAGCAACTTCAAGGAGTACCTGAGGCACGGCGAGGAGTTCGACCTGCAGTTCATCTTCCAGCTGTGCAAGATCACCCTGAGCGCCGACATCATGACCTACATCCACAGCATGAACCCCGCCATCCTGGAGGACTGGAACTTCGGCCTGACCACCCCCCCCAGCGGCAGCCTGGAGGACACCTACAGGTTCGTGACCAGCCAGGCCATCACCTGCCAGAAGTCCGCCCCCCAGAAGCCCAAGGAGGACCCCTTCAAGGACTACGTGTTCTGGGAGGTGAACCTGAAGGAGAAGTTCAGCGCCGACCTGGACCAGTTCCCCCTGGGCAGGAAGTTCCTGCTGCAGGCCGGCTACAGGGCCAGGCCCAAGTTCAAGGCCGGCAAGAGGAGCGCCCCCAGCGCCAGCACCACCACCCCGCCAAGAGGAAGAAGACCAAGAAGTAAActcgagctc |
| SEQ ID No: 35 | Synthetic HPV35L1 gene | ctgggtaccATGAGTCTGTGGAGGAGCAATGAGGCTACAGTCTACCTGCCTCCTGTGTCTGTGAGCAAGGTGGTGAGCACAGATGAATATGTGACCAGGACCAACATCTACTACCATGCTGGCTCCAGCAGACTGCTGGCTGTGGGACACCCATACTATGCCATCAAGAAGCAGGACAGCAACAAGATTGCTGTGCCAAAGGTGTCTGGACTCCAATACAGGGTGTTCAGGGTGAAACTGCCTGACCCAAACAAGTTTGGCTTTCCTGACACCTCCTTCTATGACCCTGCCAGCCAGAGACTGG |

| SEQ ID NO | Sequence description | Amino acid or nucleic acid sequence |
|---|---|---|
| | | TGTGGGCTTGTACTGGAGTGGAGGTGGGCAGGGGACAACCACTGGGAGTGGGCATCTCT GGACACCCACTGCTGAACAAACTGGATGACACAGAGAACAGCAACAAATATGTGGGCAA CTCTGGCACAGACAACAGGGAGTGTATCAGTATGGACTACAAGCAGACCCAACTTTGTCT GATTGGCTGTAGACCTCCAATTGGAGAACACTGGGGCAAGGGCACACCATGTAATGCCAA CCAGGTGAAGGCTGGAGAGTGTCCTCCATTGGAACTGCTGAACACAGTGCTCCAAGATGG AGATATGGTGGACACAGGCTTTGGAGCTATGGACTTCACCACCCTCCAAGCCAACAAGTCT GATGTGCCACTGGACATCTGTTCCAGCATCTGTAAATACCCTGACTACCTGAAAATGGTGT CTGAACCATATGGAGATATGCTGTTCTTCTACCTGAGGAGGGAACAGATGTTTGTGAGAC ACCTGTTCAACAGGGCTGGCACAGTGGGAGAGACAGTGCCTGCTGACCTCTACATCAAGG GCACCACAGGCACCCTGCCAAGCACCTCCTACTTTCCAACACCATCTGGCAGTATGGTGAC CTCTGATGCCCAGATTTTCAACAAGCCATACTGGCTCCAAAGGGCTCAAGGACACAACAAT GGCATCTGTTGGAGCAACCAACTTTTTGTGACAGTGGTGGACACCACCAGGAGCACCAAT ATGAGTGTGTGTTCTGCTGTGTCCTCCTCTGACAGCACCTACAAGAATGACAACTTCAAGG AATACCTGAGACATGGAGAGGAATATGACCTCCAATTCATCTTCCAACTTTGTAAGATTAC CCTGACAGCAGATGTGATGACCTACATCCACAGTATGAACCCAAGCATCTTGGAGGACTG GAACTTTGGACTGACACCTCCTCCATCTGGCACCTTGGAGGACACCTACAGATATGTGACC AGCCAGGCTGTGACTTGTCAGAAGCCATCTGCCCCAAAGCCAAAGGATGACCCACTGAAA AACTACACCTTCTGGGAGGTGGACCTGAAAGAGAAGTTCTCTGCTGACCTGGACCAGTTT CCACTGGGCAGGAAGTTCCTGCTCCAAGCAGGACTGAAAGCCAGACCAAACTTCAGACTG GGCAAGAGGGCTGCCCCTGCCAGCACCAGCAAGAAGTCCAGCACCAAGAGGAGGAAGGT GAAGAGCTAAActcgagctc |

| SEQ ID NO | Sequence description | Amino acid or nucleic acid sequence |
|---|---|---|
| SEQ ID No: 36 | Synthetic HPV39L1 gene | ctgggtaccATGGCTATGTGGAGGTCCTCTGACAGTATGGTCTACCTGCCTCCTCCATCTGTGGCTAAGGTGGTGAACACAGATGACTATGTGACCAGGACAGGCATCTACTACTATGCTGGCTCCAGCAGACTGCTGACAGTGGGACACCCATACTTCAAGGTGGGGATGAATGGAGGCAGGAAGCAGGACATCCCAAAGGTGTCTGCCTACCAATACAGGGTGTTCAGGGTGACCCTGCCTGACCCAAACAAGTTCAGCATCCCTGATGCCTCCCTCTACAACCCTGAGACCCAGAGACTGGTGTGGGCTTGTGTGGGAGTGGAGGTGGGCAGGGGACAACCACTGGGAGTGGGCATCTCTGGACACCCACTCTACAACAGACAGGATGACACAGAGAACAGCCCATTCTCCAGCACCACCAACAAGGACAGCAGGGACAATGTGTCTGTGGACTACAAGCAGACCCAACTTTGTATCATTGGCTGTGTGCCTGCCATTGGAGAACACTGGGGCAAGGGCAAGGCTTGTAAGCCAAACAATGTGAGCACAGGAGACTGTCCTCCATTGGAACTGGTGAACACACCAATTGAGGATGGAGATATGATTGACACAGGCTATGGAGCTATGGACTTTGGAGCCCTCCAAGAGACCAAGTCTGAGGTGCCACTGGACATCTGTCAGAGCATCTGTAAATACCCTGACTACCTCCAAATGAGTGCTGATGTCTATGGAGACAGTATGTTCTTCTGTCTGAGGAGGGAACAACTTTTTGCCAGACACTTCTGGAACAGGGGAGGGATGGTGGGAGATGCCATCCCTGCCCAACTCTACATCAAGGGCACAGACATCAGGGCTAACCCTGGCTCCTCTGTCTACTGTCCAAGCCCATCTGGCAGTATGGTGACCTCTGACAGCCAACTTTTCAACAAGCCATACTGGCTGCACAAGGCTCAAGGACAACAATGGCATCTGTTGGCACAACCAACTTTTCCTGACAGTGGTGGACACCACCAGGAGCACCAACTTCACCCTGAGCACCAGCATTGAGTCCAGCATCCCAAGCACCTATGACCCAAGCAAGTTCAAGGAATACACCAGGCATGTGGAGGAATATGACCTCCAATTCATCTTCCAACTTTGTACTGTGACCCTGACCACAGATGTGATGAGTTACATCCACACAATGAACTCCAGCATCCTGGACAACTGGAACTTTGCTGTGGCTCCTCCTCCATCTGCCTCCCTGGTGGACACCTACAGATACCTCCAATCTGCTGCCATCACTTGTCAGAAGGATGCCCCTGCCCCTGAGAAGAAGGACCCATATGATGGACTGAAGTTCTGGAATGTGGACCTGAGGGAGAAGTTCTCCTTGGAACTGGACCAGTTTCCACTGGGCAGGAAGTTCCTGCTCCAAGCCAGGGTGAGGAGGAGACCAACCATTGGACCAAGGAAGAGACCTGCTGCCAGCACCTCCTCCTCTGCCACCAAACACAAGAGGAAGAGGGTGAGCAAGTAAActcgagctc |

EP 4 292 607 A1

| SEQ ID NO | Sequence description | Amino acid or nucleic acid sequence |
|---|---|---|
| SEQ ID No: 37 | Synthetic HPV45L1 gene | ctgggtaccATGGCTCTGTGGAGACCATCTGACAGCACAGTCTACCTGCCTCCTCCATCTGTG GCAAGGGTGGTGAACACAGATGACTATGTGAGCAGGACCAGCATCTTCTACCATGCTGGC TCCAGCAGACTGCTGACAGTGGGCAACCCATACTTCAGGGTGGTGCCAAGTGGAGCAGG CAACAAGCAGGCTGTGCCAAAGGTGTCTGCCTACCAATACAGGGTGTTCAGGGTGGCTCT GCCTGACCCAAACAAGTTTGGACTGCCTGACAGCACCATCTACAACCCTGAGACCCAGAG ACTGGTGTGGGCTTGTGTGGGGATGGAGATTGGCAGGGGACAACCACTGGGCATTGGAC TGTCTGGACACCCATTCTACAACAAACTGGATGACACAGAGTCTGCCCATGCTGCCACAGC |
|  |  | AGTGATTACCCAGGATGTGAGGGACAATGTGTCTGTGGACTACAAGCAGACCCAACTTTG TATCCTGGGCTGTGTGCCTGCCATTGGAGAACACTGGGCTAAGGGCACCCTGTGTAAGCC TGCCCAACTCCAACCTGGAGACTGTCCTCCATTGGAACTGAAAAACACCATCATTGAGGAT GGAGATATGGTGGACACAGGCTATGGAGCTATGGACTTCAGCACCCTCCAAGACACCAAG TGTGAGGTGCCACTGGACATCTGTCAGAGCATCTGTAAATACCCTGACTACCTCCAAATGA GTGCTGACCCATATGGAGACAGTATGTTCTTCTGTCTGAGGAGGGAACAACTTTTTGCCAG ACACTTCTGGAACAGGGCTGGAGTGATGGGAGACACAGTGCCAACAGACCTCTACATCAA GGGCACCTCTGCCAATATGAGGGAGACACCTGGCTCCTGTGTCTACAGCCCAAGCCCATCT GGCAGCATCACCACCTCTGACAGCCAACTTTTCAACAAGCCATACGGCTGCACAAGGCTC AAGGACACAACAATGGCATCTGTTGGCACAACCAACTTTTTGTGACAGTGGTGGACACCA CCAGGAGCACCAACCTGACCCTGTGTGCCAGCACCCAGAACCCTGTGCCAAACACCTATG ACCCAACCAAGTTCAAGCACTACAGCAGGCATGTGGAGGAATATGACCTCCAATTCATCTT CCAACTTTGTACCATCACCCTGACAGCAGAGGTGATGAGTTACATCCACAGTATGAACTCC AGCATCTTGGAGAACTGGAACTTTGGAGTGCCTCCTCCTCCAACCACCTCCCTGGTGGACA CCTACAGGTTTGTCCAGTCTGTGGCTGTGACTTGTCAGAAGGACACCACACCTCCTGAGAA GCAGGACCCATATGACAAACTGAAGTTCTGGACAGTGGACCTGAAAGAGAAGTTCTCCTC TGACCTGGACCAATACCCACTGGGCAGGAAGTTCCTGGTCCAGGCTGGACTGAGGAGGA GACCAACCATTGGACCAAGGAAGAGACCTGCTGCCAGCACCAGCACAGCCAGCAGACCT GCCAAGAGGGTGAGGATTAGGAGCAAGAAGTAAA ctcgagctc |

EP 4 292 607 A1

| SEQ ID NO | Sequence description | Amino acid or nucleic acid sequence |
|---|---|---|
| SEQ ID No: 38 | Synthetic HPV51L1 gene | ctgggtaccATGGCTCTGTGGAGGACCAATGACAGCAAGGTCTACCTGCCTCCTGCCCCTGTGAGCAGGATTGTGAACACAGAGGAATACATCACCAGGACAGGCATCTACTACTATGCTGGCTCCAGCAGACTGATTACCCTGGGACACCCATACTTTCCAATCCCAAAGACCAGCACCAGGGCTGCCATCCCAAAGGTGTCTGCCTTCCAATACAGGGTGTTCAGGGTCCAACTTCCTGACCCAAACAAGTTTGGACTGCCTGACCCAAACCTCTACAACCCTGACACAGACAGACTGGTGTGGGGCTGTGTGGGAGTGGAGGTGGGCAGGGGACAACCACTGGGAGTGGGACTGTCTGGACACCCACTGTTCAACAAATATGATGACACAGAGAACAGCAGGATTGCCAATGGCAATGCCCAACAGGATGTGAGGGACAACACCTCTGTGGACAACAAGCAGACCCAACTTTGTATCATTGGCTGTGCCCCTCCAATTGGAGAACACTGGGGCATTGGCACCACTTGTAAGAACACACCTGTGCCTCCTGGAGACTGTCCTCCATTGGAACTGGTGTCCTCTGTGATTCAGGATGGAGATATGATTGACACAGGCTTTGGAGCTATGGACTTTGCTGCCCTCCAAGCCACCAAGTCTGATGTGCCACTGGACATCAGCCAGTCTGTGTGTAAATACCCTGACTACCTGAAAATGAGTGCTGACACCTATGGCAACAGTATGTTCTTCCACCTGAGGAGGGAACAGATTTTTGCCAGACACTACTACAACAAACTGGTGGGAGTGGGAGAGGACATCCCAAATGACTACTACATCAAGGGCTCTGGCAATGGCAGGGACCCAATTGAGTCCTACATCTACTCTGCCACACCATCTGGCAGTATGATTACCTCTGACAGCCAGATTTTCAACAAGCCATACTGGCTGCACAGGGCTCAAGGACACAACAATGGCATCTGTTGGAACAACCAACTTTTCATCACTTGTGTGGACACCACCAGGAGCACCAACCTGACCATCAGCACAGCCACAGCAGCAGTGAGCCCAACCTTCACACCAAGCAACTTCAAGCAATACATCAGACATGGAGAGGAATATGAACTCCAATTCATCTTCCAACTTTGTAAGATTACCCTGACCACAGAGGTGATGGCTTACCTGCACACAATGGACCCAACCATCTTGGAACAGTGGAACTTTGGACTGACCCTGCCTCCATCTGCCTCCTTGGAGGATGCCTACAGGTTTGTGAGGAATGCTGCCACCTCCTGTCAGAAGGACACACCTCCACAGGCTAAGCCTGACCCACTGGCTAAATACAAGTTCTGGGATGTGGACCTGAAAGAGAGGTTCTCCCTGGACCTGGACCAGTTTGCCCTGGGCAGGAAGTTCCTGCTCCAAGTGGGAGTCCAGAGGAAGCCAAGACCTGGACTGAAAAGACCTGCCTCCTCTGCCTCCTCCTCCTCCTCCTCTGCCAAGAGGAAGAGGGTGAAGAAGTAAActcgagctc |

| SEQ ID NO | Sequence description | Amino acid or nucleic acid sequence |
|---|---|---|
| SEQ ID No: 39 | Synthetic HPV52L1 gene | ctgggtaccATGAGCGTGTGGAGGCCCAGCGAGGCCACCGTGTACCTGCCCCCCGTGCCCGT GAGCAAGGTGGTGAGCACCGACGAGTACGTGAGCAGGACCAGCATCTACTACTACGCCG GCAGCAGCAGGCTGCTGACCGTGGGCCACCCCTACTTCAGCATCAAGAACACCAGCAGCG GCAACGGCAAGAAGGTGCTGGTGCCCAAGGTGAGCGGCCTGCAGTACAGGGTGTTCAGG ATCAAGCTGCCCGACCCCAACAAGTTCGGCTTCCCCGACACCAGCTTCTACAACCCCGAGA CCCAGAGGCTGGTGTGGGCCTGCACCGGCCTGGAGATCGGCAGGGGCCAGCCCCTGGGC GTGGGCATCAGCGGCCACCCCCTGCTGAACAAGTTCGACGACACCGAGACCAGCAACAAG TACGCCGGCAAGCCCGGCATCGACAACAGGGAGTGCCTGAGCATGGACTACAAGCAGAC CCAGCTGTGCATCCTGGGCTGCAAGCCCCCCATCGGCGAGCACTGGGGCAAGGGCACCCC |
|  |  | CTGCAACAACAACAGCGGCAACCCCGGCGACTGCCCCCCCCCTGCAGCTGATCAACAGCGT GATCCAGGACGGCGACATGGTGGACACCGGCTTCGGCTGCATGGACTTCAACACCCTGCA GGCCAGCAAGAGCGACGTGCCCATCGACATCTGCAGCAGCGTGTGCAAGTACCCCGACTA CCTGCAGATGGCCAGCGAGCCCTACGGCGACAGCCTGTTCTTCTTCCTGAGGAGGGAGCA GATGTTCGTGAGGCACTTCTTCAACAGGGCCGGCACCCTGGGCGACCCCGTGCCCGGCGA CCTGTACATCCAGGGCAGCAACAGCGGCAACACCGCCACCGTGCAGAGCAGCGCCTTCTT CCCCACCCCCAGCGGCAGCATGGTGACCAGCGAGAGCCAGCTGTTCAACAAGCCCTACTG GCTGCAGAGGGCCCAGGGCCACAACAACGGCATCTGCTGGGGCAACCAGCTGTTCGTGA CCGTGGTGGACACCACCAGGAGCACCAACATGACCCTGTGCGCCGAGGTGAAGAAGGAG AGCACCTACAAGAACGAGAACTTCAAGGAGTACCTGAGGCACGGCGAGGAGTTCGACCT GCAGTTCATCTTCCAGCTGTGCAAGATCACCCTGACCGCCGACGTGATGACCTACATCCAC AAGATGGACGCCACCATCCTGGAGGACTGGCAGTTCGGCCTGACCCCCCCCCCCCAGCGCC AGCCTGGAGGACACCTACAGGTTCGTGACCAGCACCGCCATCACCTGCCAGAAGAACACC CCCCCCAAGGGCAAGGAGGACCCCCTGAAGGACTACATGTTCTGGGAGGTGGACCTGAA GGAGAAGTTCAGCGCCGACCTGGACCAGTTCCCCCTGGGCAGGAAGTTCCTGCTGCAGGC CGGCCTGCAGGCCAGGCCCAAGCTGAAGAGGCCCGCCAGCAGCGCCCCCAGGACCAGCA CCAAGAAGAAGAAGGTGAAGAGGTAAActcgagctc |

(continued)

| SEQ ID NO | Sequence description | Amino acid or nucleic acid sequence |
|---|---|---|
| SEQ ID No: 40 | Synthetic HPV56L1 gene | ctgggtaccATGGCTACCTGGAGACCATCTGAGAACAAGGTCTACCTGCCTCCAACACCTGTGAGCAAGGTGGTGGCTACAGACTCCTATGTGAAGAGGACCAGCATCTTCTACCATGCTGGCTCCAGCAGACTGCTGGCTGTGGGACACCCATACTACTCTGTGACCAAGGACAACACCAAGACCAACATCCCAAAGGTGTCTGCCTACCAATACAGGGTGTTCAGGGTGAGACTGCCTGACCCAAACAAGTTTGGACTGCCTGACACCAACATCTACAACCCTGACCAGGAGAGACTGGTGTGGGCTTGTGTGGGATTGGAGGTGGGCAGGGGACAACCACTGGGAGCAGGACTGTCTGGACACCCACTGTTCAACAGACTGGATGACACAGAGTCCAGCAACCTGGCTAACAACAATGTGATTGAGGACAGCAGGGACAACATCTCTGTGGATGGCAAGCAGACCCAACTTTGTATTGTGGGCTGTACTCCTGCTATGGGAGAACACTGGACCAAGGGAGCAGTGTGTAAGAGCACCCAGGTGACCACAGGAGACTGTCCTCCACTGGCTCTGATAAACACACCAATTGAGGATGGAGATATGATTGACACAGGCTTTGGAGCTATGGACTTCAAGGTGCTCCAAGAGAGCAAGGCTGAGGTGCCACTGGACATTGTCCAGAGCACTTGTAAATACCCTGACTACCTGAAAATGAGTGCTGATGCCTATGGAGACAGTATGTGGTTCTACCTGAGGAGGGAACAACTTTTTGCCAGACACTACTTCAACAGGGCTGGCAAGGTGGGAGAGACCATCCCTGCTGAACTCTACCTGAAAGGCAGCAATGGCAGGGAACCTCCTCCATCCTCTGTCTATGTGGCTACACCATCTGGCAGTATGATTACCTCTGAGGCTCAACTTTTCAACAAGCCATACTGGCTCCAAAGGGCTCAAGGACACAACAATGGCATCTGTTGGGGCAACCAACTTTTTGTGACAGTGGTGGACACCACCAGGAGCACCAATATGACCATCAGCACAGCCACAGAACAACTTAGCAAATATGATGCCAGGAAGATAAACCAATACCTGAGGCATGTGGAGGAATATGAACTCCAATTTGTGTTCCAACTTTGTAAGATTACCCTGTCTGCTGAGGTGATGGCTTACCTGCACAATATGAATGCCAACCTGTTGGAGGACTGGAACATTGGACTGAGCCCTCCTGTGGCTACCTCCTTGGAGGACAAATACAGATATGTGAGGAGCACAGCCATCACTTGTCAGAGGGAACAACCTCCAACAGAGAAGCAGGACCCACTGGCTAAATACAAGTTCTGGGATGTGAACCTCCAAGACTCCTTCAGCACAGACCTGGACCAGTTTCCACTGGGCAGGAAGTTCCTGATGCAACTTGGCACCAGGAGCAAGCCTGCTGTGGCTACCAGCAAGAAGAGGTCTGCCCCAACCAGCACCAGCACACCTGCCAAGAGGAAGAGGAGGTAAActcgagctc |

EP 4 292 607 A1

(continued)

| SEQ ID NO | Sequence description | Amino acid or nucleic acid sequence |
|---|---|---|
| SEQ ID No: 41 | Synthetic HPV58L1 gene | ctgggtaccATGAGCGTGTGTGGAGGCCCAGCGAGGAGGCCACCGTGTACCTGCCCCCGTGCCCGT GAGCAAGGTGGTGAGCACCGACGAGTACGTGAGCAGGACCAGCATCTACTACGCCG GCAGCAGCAGGCTGCTGGCCGTGGGCAACCCCTACTTCAGCATCAAGAGCCCAACAACA ACAAGAAGGTGCTGGTGCCCAAGGTGAGCGGCCTGCAGTACAGGGTGTTCAGGGTGAGG CTGCCCGACCCCAACAAGTTCGGCTTCCCCGACACCAGCTTCTACAACCCCGACACCCAGA GGCTGGTGTGGGCCTGGCGTGGGCCTGGAGATCGGCAGGGGCCAGCCCCTGGGCGTGGG CGTGAGCGGCCACCCCTACCTGAACAAGTTCGACGACGACCAGACCAGCAACAGGTACCC CGCCCAGCCCGGCAGCGACAACAGGGAGGTGCCTGAGCATGGACTACAAGCAGACCCAGC TGTGCCTGATCGGCTGCAAGCCCCCCACCGGACTGCCCCCCCTGGAGCTGTTCAACAGCATCATGAG AACAACAACGCCGCCGCCCACCGACTGCCCCCCGTGCCCGAGCACGGGCAAGGGCGTGGCCTGC GACGGCGACATGGTGGACACCGGCTTCGGCTGCATGGACTTCGGCACCCTGCAGGCCAAC AAGAGCGACGTGCCCATCGACATCTGCAACAGCACCTGCAAGTACCCCGACTACCTGAAG ATGGCCGACGAGCCCTACGGCGACAGCCTGTTCTTCTTCCTGAGGAGGGAGCAGATGTTC GTGAGGCACTTCTTCAACAGGGCCGGCAAGCTGGGCGAGGCCGTGCCCGACGACCTGTA CATCAAGGGCAGCGGCAACACCGCCGTGATCCAGAGCAGCGCCTTCTTCCCCACCCCAG CGGCAGCATCGTGACCAGCGAGAGCCAGCTGCTGGGCAACCAGCTGTTCGTGACCGTGGTGGACA CCCAGGGCCACAACAACGGCATCTGCTGGGGCAACCAGCTGTTCGTGACCGTGGTGGACA CCACCAGGAGCACCAACATGACCCTGTGCACCGAGGTGACCAAGGAGGGCACCTACAAG AACGACAACTTCAAGGAGTACGTGAGGCACGTGGAGGAGTACGACCTGCAGTTCGTGTTC CAGCTGTGCAAGATCACCCTGAGCGCCGAGATCATGACCTACATCACACATGGACAGC AACATCCTGGAGGACTGGCAGTTCGGCCTGCAGCCCATCACCTGCCAGAAGACCGCCCCCAAGGAG ACCTACAGGTTCGTGACCAGCCAGGCTACAACCTTCTGGGAGGTGAACCTGAAGGAGAAGTTCAG AAGGAGGACCCCCTGAACAAGTACACACCTTCTGGGAGGTGAACCTGAAGGAGAAGTTCAG CGCCGACCTGGACCAGTTCCCCCTGGGCAGGAAGTTCTGCTGCGAGAGCGGCCTGAAGGC CAAGCCCAGGCTGAAGAAGAAGTAAG AGGTGAAGAAGTAAA ctcgagctc |

**Claims**

1. A stable formulation of a multivalent human papillomavirus virus-like particle vaccine for the prevention of HPV-related diseases or infections comprising

    (a) a plurality of human papillomavirus virus-like particles;
    (b) an adjuvant;
    (c) a physiologically acceptable concentration of a buffer;
    (d) a physiologically acceptable concentration of an osmotic pressure regulator; and optionally
    (e) a physiologically acceptable concentration of a surfactant,
    wherein the human papillomavirus virus-like particles are selected from HPV virus-like particles assembled by L1 proteins of HPV types 6, 11, 16, 18, 31, 33, 45, 52 and 58; and
    one or more HPV virus-like particles assembled from L1 proteins of other pathogenic HPV types,
    wherein that human papillomavirus virus-like particle are adsorbed on an adjuvant.

2. The formulation according to claim 1, wherein

    the buffer is selected from one or more of citric acid buffer, acetic acid buffer or histidine buffer;
    the osmotic pressure regulator is selected from one or more of sodium chloride, sodium phosphate or sodium sulfate;
    the surfactant is a polyethoxy ether, preferably polysorbate 80;
    the adjuvant is preferably an aluminum adjuvant, more preferably one or more of aluminum hydroxyphosphate ($AlPO_4$), amorphous aluminum hydroxyphosphate sulfate (AAHS) or aluminum hydroxide (Al $(OH)_3$), most preferably aluminum hydroxyphosphate ($AlPO_4$).

3. The formulation according to claim 1 or 2, wherein

    (a) the total concentration of papillomavirus virus-like particles of all types is 40 to 740 $\mu$ g/mL;
    (b) the concentration of the buffer is 10mM to 26 mM, preferably 10 mM, 18mM or 26 mM;
    (c) the concentration of the osmotic pressure regulator is 150mM to 320 mM, preferably 150 mM or 320 mM;
    (d) the concentration of the surfactant is 0 to 0.02 wt%;
    (e) the concentration of the adjuvant is about 1.0mg/mL;
    (f) the pH of the formulation is from 5.9 to 6.5, preferably 5.9, 6.2 or 6.5.

4. The formulation according to claim 3, wherein the concentration of any single type of papillomavirus virus-like particles included in the multivalent papillomavirus virus-like particles is 40 $\mu$g/mL to 120 $\mu$g/mL.

5. The formulation according to any of claims 1-4, comprising 0.74mg/mL of the total papillomavirus virus-like particles of all types , 1.0mg/mL of aluminum phosphate adjuvant, 18mM histidine buffer, 320mM sodium chloride, a pH of 6.2;and

    optionally, polysorbate 80 at a concentration of not more than 0.3mg/mL.

6. The formulation according to claim 5, wherein the one or more other pathogenic HPV types are selected from HPV type 35, 39, 51, 56 and 59.

7. The formulation according to claim 6, wherein at least one of the HPV virus-like particles is a chimeric HPV virus-like particle comprising a chimeric HPV L1 protein; the chimeric HPV L1 protein comprises from that N-terminal to the C-terminal thereof:

    a. an N-terminal fragment derived from L1 protein of the first papilloma virus type, said N-terminal fragment maintains the immunogenicity of the L1 protein of the first papilloma virus type, wherein said L1 protein of the first papilloma virus type is selected from HPV Types 6, 11, 16, 18, 31, 35, 39, 45, 51, 52, 56, 58, and one or more other pathogenic HPV types; and
    b. a C-terminal fragment derived from L1 protein of the second papilloma virus type, said L1 protein of the second papilloma virus type has a better expression level and solubility compared to L1 proteins of other types;

    wherein the chimeric HPV L1 protein has the immunogenicity of the L1 protein of the first papilloma virus type.

8. The formulation according to claim 7, wherein

said N-terminal fragment is a fragment obtained by truncating the C-terminus of the natural sequence of said L1 protein of the first papilloma virus type at any amino acid position within its α5 region, and a fragment having at least 98% identity therewith; and

said C-terminal fragment is a fragment obtained by truncating the N-terminus of the natural sequence of said L1 protein of the second papilloma virus type at any amino acid position within its α5 region and functional variants resulting from further mutations, deletions and/or additions to the fragment.

9. The formulation according to claim 8, wherein the C-terminal fragment comprises one or more nuclear localization sequences.

10. The formulation according to claim 7,

wherein the papilloma L1 protein of the first type is selected from HPV type 6, 11, 16, 18, 31, 35, 39, 45, 51, 52, 56 or 58; preferably, the natural sequence thereof is an amino acid sequence encoded by a coding gene shown in SEQ ID No: 30, SEQ ID No: 31, SEQ ID No: 32, SEQ ID No: 33, SEQ ID No: 34, SEQ ID No: 35, SEQ ID No: 36, SEQ ID No: 37, SEQ ID No: 38, SEQ ID No: 39, SEQ ID No: 40, or SEQ ID No: 41, respectively;

wherein that papillomavirus L1 protein of the second type is selected from HPV type 16, 28, 33, 59 or 68 L1 protein; more preferably, the papilloma L1 protein of the second type is selected from the group consisting of an HPV type 33 or 59 L1 protein.

11. The formulation according to claim 10, wherein the C-terminal fragment is SEQ ID No: 1; or a fragment thereof having a length of m1 amino acids, preferably a fragment covering amino acids 1-m1 of SEQ ID No: 1; wherein m1 is an integer from 8 to 26; or that C-terminal fragment is SEQ ID No: 2; or fragments thereof having a length of m2 amino acids,

preferably a fragment comprising amino acids 1-m2 of SEQ ID No: 2; wherein m2 is an integer from 13 to 31.

12. The formulation according to claim 10, wherein the C-terminal fragment is SEQ ID No: 3; or a fragment thereof having a length of n amino acids, preferably a fragment covering amino acids 1-n of SEQ ID No: 3; wherein n is an integer from 16 to 38.

13. The formulation according to claim 7, wherein

the N-terminal fragment of the HPV type 6 L1 protein has 98%, 98.5%, 99%, 99.5%, 99% or 100% identity with a fragment obtained by truncating the C-terminal of the sequence shown in SEQ ID No:4 at any amino acid site within the α5 region thereof;

the N-terminal fragment of the HPV type 11 L1 protein has 98%, 98.5%, 99%, 99.5%, 99% or 100% identity with a fragment obtained by truncating the C-terminal of the sequence shown in SEQ ID No:5 at any amino acid site within the α5 region thereof;

the N-terminal fragment of the HPV type 16 L1 protein has 98%, 98.5%, 99%, 99.5%, 99% or 100% identity with a fragment obtained by truncating the C-terminal of the sequence shown in SEQ ID No:6 at any amino acid site within the α5 region thereof;

the N-terminal fragment of the HPV type 18 L1 protein has 98%, 98.5%, 99%, 99.5%, 99% or 100% identity with a fragment obtained by truncating the C-terminal of the sequence shown in SEQ ID No: 7 at any amino acid site within the α5 region thereof;

the N-terminal fragment of the HPV type 31 L1 protein has 98%, 98.5%, 99%, 99.5%, 99% or 100% identity with a fragment obtained by truncating the C-terminal of the sequence shown in SEQ ID No:8 at any amino acid site within the α5 region thereof;

the N-terminal fragment of the HPV type 35 L1 protein has 98%, 98.5%, 99%, 99.5%, 99% or 100% identity with a fragment obtained by truncating the C-terminal of the sequence shown in SEQ ID No: 9 at any amino acid site within the α5 region thereof;

the N-terminal fragment of the HPV type 39 L1 protein has 98%, 98.5%, 99%, 99.5%, 99% or 100% identity with a fragment obtained by truncating the C-terminal of the sequence shown in SEQ ID No: 10 at any amino acid site within the α5 region thereof;

the N-terminal fragment of the HPV type 45 L1 protein has 98%, 98.5%, 99%, 99.5%, 99% or 100% identity with a fragment obtained by truncating the C-terminal of the sequence shown in SEQ ID No: 11 at any amino acid site within the α5 region thereof;

the N-terminal fragment of the HPV type 51 L1 protein has 98%, 98.5%, 99%, 99.5%, 99% or 100% identity with a fragment obtained by truncating the C-terminal of the sequence shown in SEQ ID No: 12 at any amino acid site within the α5 region thereof;

the N-terminal fragment of the HPV type 52 L1 protein has 98%, 98.5%, 99%, 99.5%, 99% or 100% identity with a fragment obtained by truncating the C-terminal of the sequence shown in SEQ ID No:13 to any amino acid site in the α5 region;

the N-terminal fragment of the HPV type 56 L1 protein has 98%, 98.5%, 99%, 99.5%, 99% or 100% identity with a fragment obtained by truncating the C-terminal of the sequence shown in SEQ ID No:14 to any amino acid site in the α5 region; and

the N-terminal fragment of the HPV type 58 L1 protein has 98%, 98.5%, 99%, 99.5%, 99% or 100% identity with a fragment obtained by truncating the C-terminal of the sequence shown in SEQ ID No: 15 at any amino acid site within the α5 region thereof.

14. The formulation according to claim 7, wherein the C-terminal of the N-terminal fragment is connected directly to the N-terminal of the C-terminal fragment or by a linker.

15. The formulation according to claim 7, wherein when the C-terminus of said N-terminal fragment is connected to the N-terminus of said C-terminal fragment, the continuous amino acid sequence RKFL is present within a range of plus or minus 4 amino acid positions of the splicing site,

preferably, the continuous amino acid sequence LGRKFL is present within a range of plus or minus 6 amino acid positions of the splicing site.

16. The formulation according to claim 7, wherein

the chimeric HPV L1 protein of type 6, 11, 16, 18, 31, 35, 39, 45, 51, 52, 56, and 58 have 98%, 98.5%, 99%, 99.5% or 100% identity to SEQ ID No:16, SEQ ID No:17, SEQ ID No:18, SEQ ID No:19, SEQ ID No:20, SEQ ID No:21, SEQ ID No:22, SEQ ID No:23, SEQ ID No:24, SEQ ID No:25, SEQ ID No:26 and SEQ ID No: 27, respectively; and

the HPV type 33 L1 protein and the HPV type 59 L1 protein have 98%, 98.5%, 99%, 99.5%, or 100% identity to SEQ ID No:28 and SEQ ID No: 29, respectively.

17. The formulation according to claim 16, comprising

HPV type 6, 11, 16, 18, 31, 35, 39, 45, 51, 52, 56 and 58 chimeric HPV L1 protein having the amino acid sequences shown in SEQ ID No:16, SEQ ID No:17, SEQ ID No:18, SEQ ID No:19, SEQ ID No:20, SEQ ID No:21, SEQ ID No:22, SEQ ID No:23, SEQ ID No:24, SEQ ID No:25, SEQ ID No:26 and SEQ ID No: 27, respectively; and

HPV type 33 L1 protein and HPV type 59 L1 protein having the amino acid sequences shown in SEQ ID No:28 and SEQ ID No: 29, respectively.

18. The formulation of a papilloma virus vaccine according to claims 1 -17, **characterized in that** it can be stably stored at 2 to 8 °C for at least 24 months and at 25 °C for at least 16 weeks.

19. A method of preventing an HPV-related disease or infection comprising:
administering the formulation according to any of claims 1-18 to a subject.

20. Use of the formulation according to any of claims 1-18 in the formulation of a vaccine for the prevention of HPV-related diseases or infections.

Figure 1

Figure 2

Figure 3

Figure 4

Figure 5

Figure 6

Figure 7

Figure 8

## INTERNATIONAL SEARCH REPORT

| | |
|---|---|
| International application No. | |
| | **PCT/CN2022/071782** |

**A. CLASSIFICATION OF SUBJECT MATTER**

A61K 39/12(2006.01)i; A61K 39/295(2006.01)i; A61K 39/39(2006.01)i; A61P 31/20(2006.01)i; A61P 35/00(2006.01)i; A61P 15/00(2006.01)i

According to International Patent Classification (IPC) or to both national classification and IPC

**B. FIELDS SEARCHED**

Minimum documentation searched (classification system followed by classification symbols)

A61K,A61P

Documentation searched other than minimum documentation to the extent that such documents are included in the fields searched

Electronic data base consulted during the international search (name of data base and, where practicable, search terms used)

CNTXT, VEN, DWPI, 超星读秀学术, CHAOXING DUXIU SCHOLAR, 中国专利生物序列检索系统, Chinese Patent Biological Sequence Retrieval System, CNKI, 万方, WANFANG, ISI web of knowledge, Elsevier Science Direct, Springerlink, pubmed, genbank, ENA, EMBL, Uniport, STN, 百度, BAIDU, 百度学术, BAIDU SCHOLAR, patentics: 神州细胞工程, SINOCELL, 刘艳, LIU YAN, 胡萍, HU PING, 常欣荣, CHANG XINRONG, HPV, 人乳头瘤病毒, human papillomavirus, L1 蛋白, L1 protein, 病毒样颗粒, VLP, 嵌合, chimeric, 杂合, Hybrid, 佐剂, adjuvant, 铝佐剂, 缓冲液, buffer, 渗透压调节剂, Osmotic pressure regulator, 氯化钠, NaCl, 表面活性剂, Surfactant, 聚山梨酯80, Polysorbate, 可溶性, soluble, Solubility, 核定位, nuclear localization, 稳定, stability, RKFL, LGRKFL, SEQ ID NOs: 1-41

**C. DOCUMENTS CONSIDERED TO BE RELEVANT**

| Category* | Citation of document, with indication, where appropriate, of the relevant passages | Relevant to claim No. |
|---|---|---|
| X | CN 102349996 A (SHENYANG SUNSHINE PHARMACEUTICAL CO., LTD.) 15 February 2012 (2012-02-15)<br>  claims 1-10, and description, paragraphs 2-4, 12-13, 21, and 34-35 | 1-6, 18-20 |
| Y | CN 102349996 A (SHENYANG SUNSHINE PHARMACEUTICAL CO., LTD.) 15 February 2012 (2012-02-15)<br>  claims 1-10, and description, paragraphs 2-4, 12-13, 21, and 34-35 | 7-20 |
| Y | CHRISTENSEN, N. D. et al. "Hybrid papillomavirus L1 molecules assemble into virus-like particles that reconstitute conformational epitopes and induce neutralizing antibodies to distinct HPV types."<br>*VIROLOGY.*, Vol. 291, No. 2, 20 December 2001 (2001-12-20),<br>  pp. 324-334 | 7-20 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **28 March 2022** | **14 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

# EP 4 292 607 A1

<table>
<tr><td colspan="2" align="center"><strong>INTERNATIONAL SEARCH REPORT</strong></td><td>International application No.<br><br><strong>PCT/CN2022/071782</strong></td></tr>
</table>

**C.     DOCUMENTS CONSIDERED TO BE RELEVANT**

| Category* | Citation of document, with indication, where appropriate, of the relevant passages | Relevant to claim No. |
|---|---|---|
| Y | 金石 (JIN, Shi). "人乳头瘤病毒(HPV)衣壳蛋白L1的体外可控组装 (Controlled Assembly of Human Papillomavirus Capsid Protein L1 in Vitro)" <br> 中国博士学位论文全文数据库医药卫生科技辑 *(China Doctoral Dissertations Full-text Database, Medicine and Health Sciences)*, 15 January 2015 (2015-01-15), <br> pp. E072-205 | 7-20 |
| PY | WO 2021013060 A1 (SINOCELLTECH LTD.) 28 January 2021 (2021-01-28) <br> claims 1-23 | 7-20 |
| PY | WO 2021013062 A1 (SINOCELLTECH LTD.) 28 January 2021 (2021-01-28) <br> claims 1-23 | 7-20 |
| PY | WO 2021013063 A1 (SINOCELLTECH LTD.) 28 January 2021 (2021-01-28) <br> claims 1-23 | 7-20 |
| PY | WO 2021013067 A1 (SINOCELLTECH LTD.) 28 January 2021 (2021-01-28) <br> claims 1-23 | 7-20 |
| PY | WO 2021013069 A1 (SINOCELLTECH LTD.) 28 January 2021 (2021-01-28) <br> claims 1-23 | 7-20 |
| PY | WO 2021013070 A1 (SINOCELLTECH LTD.) 28 January 2021 (2021-01-28) <br> claims 1-23 | 7-20 |
| PY | WO 2021013071 A1 (SINOCELLTECH LTD.) 28 January 2021 (2021-01-28) <br> claims 1-17 | 7-20 |
| PY | WO 2021013072 A1 (SINOCELLTECH LTD.) 28 January 2021 (2021-01-28) <br> claims 1-23 | 7-20 |
| PY | WO 2021013073 A1 (SINOCELLTECH LTD.) 28 January 2021 (2021-01-28) <br> claims 1-20 | 7-20 |
| PY | WO 2021013075 A1 (SINOCELLTECH LTD.) 28 January 2021 (2021-01-28) <br> claims 1-23 | 7-20 |
| PY | WO 2021013076 A1 (SINOCELLTECH LTD.) 28 January 2021 (2021-01-28) <br> claims 1-23 | 7-20 |
| PY | WO 2021013077 A1 (SINOCELLTECH LTD.) 28 January 2021 (2021-01-28) <br> claims 1-23 | 7-20 |
| PY | WO 2021013078 A1 (SINOCELLTECH LTD.) 28 January 2021 (2021-01-28) <br> claims 1-23 | 7-20 |
| PY | WO 2021013079 A1 (SINOCELLTECH LTD.) 28 January 2021 (2021-01-28) <br> claims 1-23 | 7-20 |
| A | WO 0197840 A1 (MEDIMMUNE, INC.) 27 December 2001 (2001-12-27) <br> entire document | 1-20 |
| A | CN 101245099 A (MA, Runlin et al.) 20 August 2008 (2008-08-20) <br> entire document | 1-20 |
| A | LI, Z. H. et al. "Rational design of a triple-type human papillomavirus vaccine by compromising viral-type specificity." <br> *NAT COMMUN.*, Vol. 9, No. 1, 18 December 2018 (2018-12-18), <br> article number: 5360 | 1-20 |
| A | PIMIENTA, E. et al. "Cloning and expression in Escherichia coli of the full-lengh and deletion variants of a human papillomavirus 18 L1 gene isolated from a Cuban patient." <br> *REV CUBANA MED TROP 2019.*, Vol. 71, No. 2, 02 November 2019 (2019-11-02), <br> article number: e301 | 1-20 |

Form PCT/ISA/210 (second sheet) (January 2015)

## INTERNATIONAL SEARCH REPORT

| International application No. |
|---|
| **PCT/CN2022/071782** |

| **Box No. I**      **Nucleotide and/or amino acid sequence(s) (Continuation of item 1.c of the first sheet)** |
|---|

1. With regard to any nucleotide and/or amino acid sequence disclosed in the international application, the international search was carried out on the basis of a sequence listing:

     a.   ☑ forming part of the international application as filed:

         ☑ in the form of an Annex C/ST.25 text file.

         ☐ on paper or in the form of an image file.

     b.   ☐ furnished together with the international application under PCT Rule 13*ter*.1(a) for the purposes of international search only in the form of an Annex C/ST.25 text file.

     c.   ☐ furnished subsequent to the international filing date for the purposes of international search only:

         ☐ in the form of an Annex C/ST.25 text file (Rule 13*ter*.1(a)).

         ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT

| International application No. |
| --- |
| **PCT/CN2022/071782** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **19**
   because they relate to subject matter not required to be searched by this Authority, namely:

   [1]  The subject matter set forth in claim 19 relates to a method for treating a human body or animal body. Therefore, claim 19 falls within the subject matter as defined in PCT Rule 39.1 that does not warrant a search conducted by the international searching authority. Nevertheless, a search is still performed on the basis of a pharmaceutical use of the formulation.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

International application No.

**PCT/CN2022/071782**

| Patent document cited in search report | | | Publication date (day/month/year) | Patent family member(s) | | | Publication date (day/month/year) |
|---|---|---|---|---|---|---|---|
| CN | 102349996 | A | 15 February 2012 | None | | | |
| WO | 2021013060 | A1 | 28 January 2021 | None | | | |
| WO | 2021013062 | A1 | 28 January 2021 | None | | | |
| WO | 2021013063 | A1 | 28 January 2021 | None | | | |
| WO | 2021013067 | A1 | 28 January 2021 | None | | | |
| WO | 2021013069 | A1 | 28 January 2021 | None | | | |
| WO | 2021013070 | A1 | 28 January 2021 | None | | | |
| WO | 2021013071 | A1 | 28 January 2021 | None | | | |
| WO | 2021013072 | A1 | 28 January 2021 | None | | | |
| WO | 2021013073 | A1 | 28 January 2021 | AU | 2020318114 | A1 | 10 February 2022 |
| WO | 2021013075 | A1 | 28 January 2021 | None | | | |
| WO | 2021013076 | A1 | 28 January 2021 | None | | | |
| WO | 2021013077 | A1 | 28 January 2021 | None | | | |
| WO | 2021013078 | A1 | 28 January 2021 | None | | | |
| WO | 2021013079 | A1 | 28 January 2021 | None | | | |
| WO | 0197840 | A1 | 27 December 2001 | US | 2006127979 | A1 | 15 June 2006 |
| | | | | PT | 1292328 | E | 19 May 2008 |
| | | | | CY | 1108106 | T1 | 12 February 2014 |
| | | | | AU | 2001275458 | B2 | 19 August 2004 |
| | | | | AT | 386105 | T | 15 March 2008 |
| | | | | CA | 2411945 | A1 | 27 December 2001 |
| | | | | DK | 1292328 | T3 | 16 June 2008 |
| | | | | AU | 7545801 | A | 02 January 2002 |
| | | | | DE | 60132770 | D1 | 27 March 2008 |
| | | | | US | 2004224305 | A1 | 11 November 2004 |
| | | | | EP | 1292328 | A1 | 19 March 2003 |
| | | | | ES | 2301551 | T3 | 01 July 2008 |
| CN | 101245099 | A | 20 August 2008 | WO | 2008098484 | A1 | 21 August 2008 |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110049777

- CN 2020102601 W

**Non-patent literature cited in the description**

- **M. SHANK-RETZLAFF ; F. WANG ; T. MORLEY et al.** Correlation between Mouse Potency and In Vitro Relative Potency for Human Papillomavirus Type 16 Virus-Like Particles and Gardasil Vaccine Samples. *Human Vaccines,* vol. 1 (5), 191-197
- **MICHAEL J. CAULFIELD ; LI SHI ; SU WANG et al.** Effect of Alternative Aluminum Adjuvants on the Absorption and Immunogenicity of HPV16 L1 VLPs in Mice. *Human Vaccines,* vol. 3 (4), 139-146

- **M. SHANK-RETZLAFF ; F. WANG ; T. MORLEY et al.** Correlation between Mouse Potency and In Vitro Relative Potency for Human Papillomavirus Type 16 Virus-Like Particles and Gardasil Vaccine Samples. *Human Vaccines,* vol. 1 (5), 191-197